# EUROPEAN PATENT APPLICATION

(11) **EP 4 043 013 A1**
(43) Date of publication of application: **17.08.2022**
(21) Application number: 20869531.2
(22) Date of filing: 27.09.2020
(51) Int. Cl.: A61K 31/285, A61K 31/7052, A61K 39/395, A61P 25/18, A61P 25/22, A61P 25/24

(54) **METHOD FOR TREATING MOOD DISORDERS**

(30) Priority: 27.09.2019 WO PCT/CN2019/108548; 23.09.2020 CN 202011009274
(71) Applicant: Jiangsu Nuo-Beta Pharmaceutical Technology Co., Ltd., Natong, Jiangsu 226499 (CN)
(72) Inventor: HUANG, Fude, Shanghai 201199 (CN); WANG, Wen'an, Shanghai 200333 (CN); JIAO, Changping, Shanghai 201299 (CN); HUANG, Changde, Shanghai 201199 (CN); HUANG, Tide, Shanghai 201199 (CN)
(74) Representative: Lahrtz, Fritz
(86) International application number: PCT/CN2020/118044
(87) International publication number: WO 2021/057955

(57) **Abstract**

Provided are an inhibitor of PI4KIIIα/FAM126/TTC7 complex and a method that uses the inhibitor to prevent or treat mood disorders.

SEQUENCE LISTING

<110>  JIANGSU MUO-BETA PHARMACEUTICAL TECHNOLOGY CO., LTD.

<120>  METHOD FOR TREATING MOOD DISORDERS

<130>  J74183PCEP

<160>  5

<170>  PatentIn version 3.5

<210>  1
<211>  19
<212>  DNA
<213>  Artificial Sequence

<220>
<223>  synthetic

<400>  1
tgctcattag cagtaaaga                                                    19


<210>  2
<211>  20
<212>  DNA
<213>  Artificial Sequence

<220>
<223>  synthetic

<400>  2
gtaccaccat gtacccaggc                                                   20


<210>  3
<211>  21
<212>  DNA
<213>  Artificial Sequence

<220>
<223>  synthetic

<400>  3
aacgcagctc agtaacagtc c                                                 21


<210>  4
<211>  20
<212>  DNA
<213>  Artificial Sequence

<220>
<223>  synthetic

<400>  4
cctagcctag cacctgaagc                                                   20


<210>  5
<211>  19
<212>  DNA

<213>  Artificial Sequence

<220>
<223>  synthetic

<400>  5
cgtgtgcatc aacggtcca                                                    19

## Description

### FIELD OF THE INVENTION

The present invention relates to a method for preventing or treating a mood disorder, including administering an effective amount of PI4KIIIα/FAM126/TTC7 complex inhibitor to a subject in need thereof.

### BACKGROUND

A mood disorder is a psychiatric disorder represented by anxiety, depression, or bipolar disorder, which tends to increase in recent years and becomes a major social problem. Anxiety and depression are the most common psychiatric disorders and are complications of other neuropsychiatric diseases, strokes, tumors, and the like. Over 70% of people with dementia have symptoms of depression and/or anxiety (Selbaek, G., K. Engedal and S. Bergh, The prevalence and course of neuropsychiatric symptoms in nursing home patients with dementia: a systematic review. J Am Med Dir Assoc, 2013. 14(3): p. 161-9).

At present, clinical drugs for anti-anxiety or anti-depression have a generally unsatisfactory effect, and particularly have poor or substantially no efficacy on severe anxiety or depression, depression caused by a neurodegenerative disease or stroke, and anxiety or depression of patients with a malignant tumor, and also have the disadvantages of slow onset of action, and extensive and serious side effects.

Prior to the present application, it was generally considered that the decreased level of phosphoinositide (PI) and decreased activity of phosphoinositide 3-kinase (PI3K) are associated with depression. PI3K deficiency increases the level of anxiety of mice, and several types of conventional antidepressants contribute to the resynthesis of PI associated with anxiety and depression in the brain (referring to Di Paolo, G. and P. De Camilli, Phosphoinositides in cell regulation and membrane dynamics. Nature, 2006. 443(7112): p. 651-7; Saito, T., et al., Polymorphism screening of PIK4CA: possible candidate gene for chromosome 22q11-linked psychiatric disorders. Am J Med Genet B Neuropsychiatr Genet, 2003. 116B(1): p. 77-83; Balla, A. and T. Balla, Phosphatidylinositol 4-kinases: old enzymes with emerging functions. Trends Cell Biol, 2006. 16(7): p. 351-61).

Surprisingly, it was found by the present application that, for mice models with various diseases, the reduction in the expression of PI4KIIIα or in the expression of Efr3a for anchoring a PI4KIIIα/FAM126/TTC7 complex by a genetic method, or the application of a PI4KIIIα inhibitor, can reduce the degree of anxiety or depression of mice. Therefore, the present application provides a potential anti-mood disorder target (that is, a PI4KIIIα protein complex) and a series of potential drugs for treating a mood disorder.

### SUMMARY

According to an aspect, the present application provides a method for preventing or treating a mood disorder, including administering an effective amount of PI4KIIIα/FAM126/TTC7 complex inhibitor to a subject in need thereof.

In some embodiments, the PI4KIIIα/FAM126/TTC7 complex inhibitor is a small-molecule compound, an antibody, an RNAi molecule, or an antisense nucleic acid.

In some embodiments, the PI4KIIIα/FAM126/TTC7 complex inhibitor is an EFR3-specific inhibitor.

In some embodiments, the EFR3-specific inhibitor is an EFR3a-specific inhibitor or an EFR3b-specific inhibitor.

In some embodiments, the PI4KIIIα/FAM126/TTC7 complex inhibitor is a PI4KIIIα-specific inhibitor.

In some embodiments, the PI4KIIIα-specific inhibitor is a small-molecule compound.

In some embodiments, the small-molecule compound has a structure of formula (I) or a pharmaceutically acceptable salt thereof, 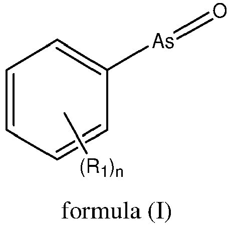 wherein R₁ is independently selected from (a) H, deuterium, halogen, nitro, cyano, hydroxyl, amino, carbamoyl, C₁₋₆ alkyl, C₂₋₆ alkynyl, C₂₋₆ alkenyl, C₁₋₆ alkoxy, C₁₋₆ haloalkyl, C₁₋₆ alkylene-NH₂, C₁₋₆ alkylene-NH-C(O)H, -As(O), -N=NH, -NH-(C₁₋₆ alkyl), - N,N-(C₁₋₆ alkyl)₂, -NH-C(O)H, -NH-S(O)₂H, -C(O)OH, -OC(O)H, -SH, -S(O)₂H, -S(O)₂-NH, or heterocyclyl, which is optionally substituted by R₂ or R₃, wherein R₂ and R₃ are each independently selected from amino, C₁₋₆ alkyl, C₁₋₆ alkoxy, C₁₋₆ haloalkyl, -NH-(C₁₋₆ alkyl), - NH-(6- to 12-membered aryl), -N,N-(C₁₋₆ alkyl)₂, C₃₋₆ cycloalkyl, 6- to 12-membered aryl, or 3- to 12-membered heterocyclyl, which is optionally substituted by one or more of halogen, nitro, cyano, hydroxyl, amino, carbamoyl, -NH-C(O)-R₅, -C(O)OR₄, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-, and R₄ is C₁₋₆ alkyl, which is optionally substituted by one or more of halogen, nitro, cyano, hydroxyl, amino, carbamoyl, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-, R₅ is selected from H, C₁₋₆ alkyl, C₂₋₆ alkynyl, C₂₋₆ alkenyl, C₁₋₆ alkoxy, or C₁₋₆ haloalkyl, or
(b) R₁ on two adjacent carbon atoms forms 5- to 12-membered cycloalkyl, aryl, or heterocyclyl, which is optionally substituted by one or more of halogen, nitro, cyano, hydroxyl, amino, carbamoyl, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-,

Wherein n is an integer from 0 to 5.

In some embodiments, R₁ is independently selected from H, deuterium, halogen, nitro, cyano, hydroxyl, amino, carbamoyl, C₁₋₆ alkyl, C₁₋₆ alkoxy, C₁₋₆ haloalkyl, - As(O), -NH-(C₁₋₆ alkyl), -N,N-(C₁₋₆ alkyl)₂, or -C(O)OR₆, wherein n is an integer from 0 to 2, and R₆ is C₁₋₆ alkyl.

In some embodiments, R₁ is independently selected from H, deuterium, halogen, nitro, cyano, hydroxyl, amino, C₁₋₆ alkyl, C₁₋₆ alkoxy, C₁₋₆ haloalkyl, or -As(O), wherein n is an integer from 0 to 2.

In some embodiments, R₁ is independently selected from H, deuterium, halogen, amino, C₁₋₆ alkoxy, or C₁₋₆ haloalkyl, wherein n is 1.

In some embodiments, R₁ is located at an ortho position and/or para position of the -As(O) group.

In some embodiments, n is 0.

In some embodiments, the small-molecule compound is selected from the group consisting of the following compounds:

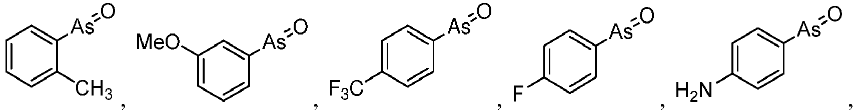

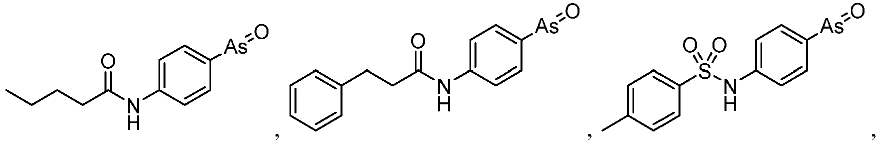

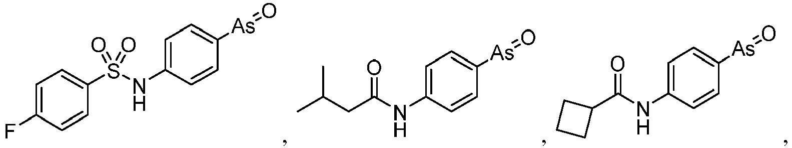

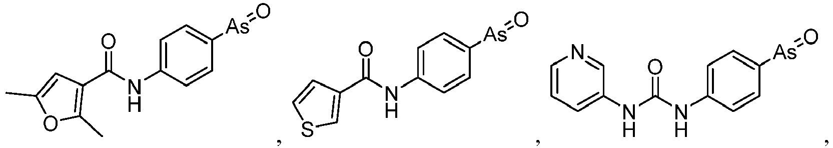

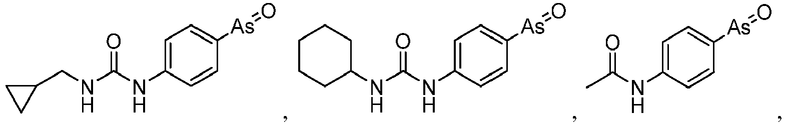

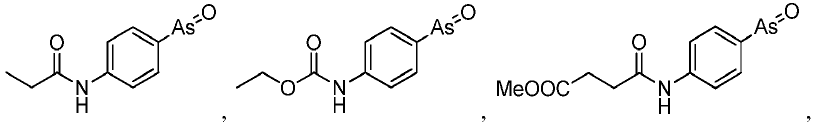

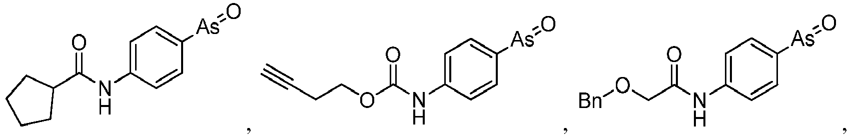

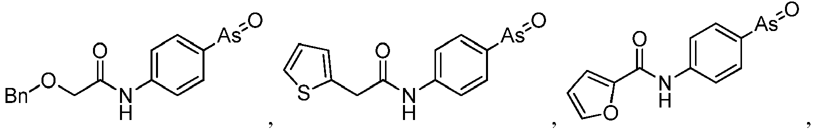

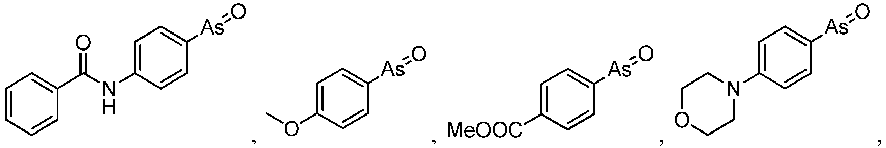

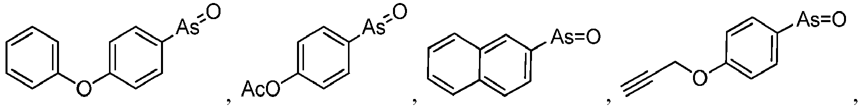

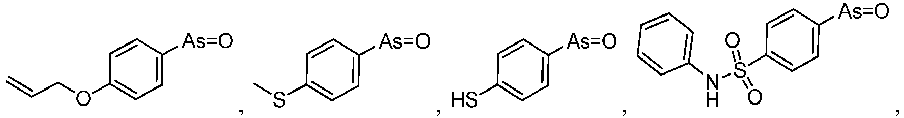

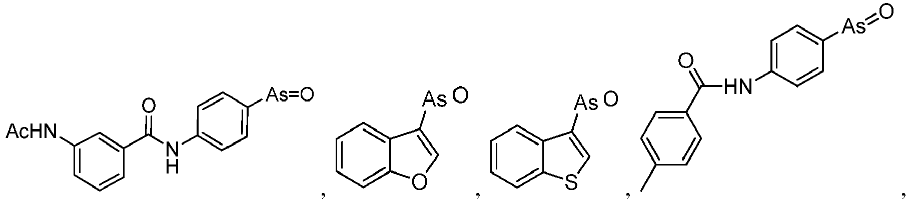

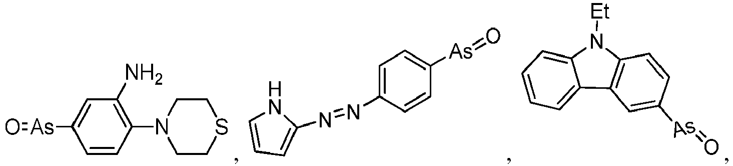

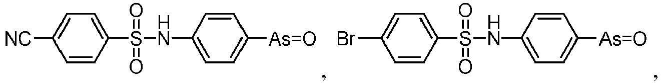

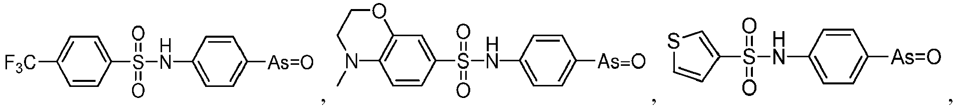

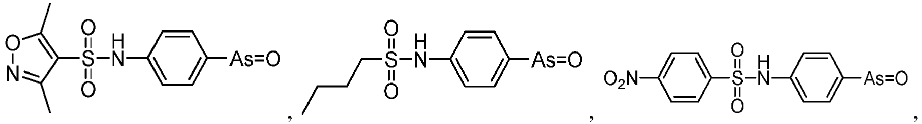

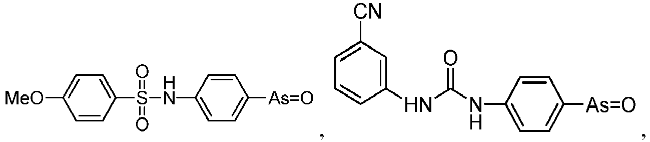

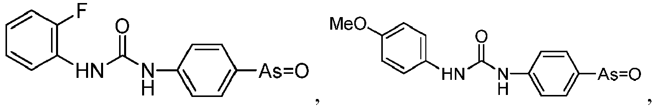

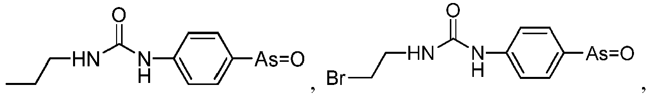

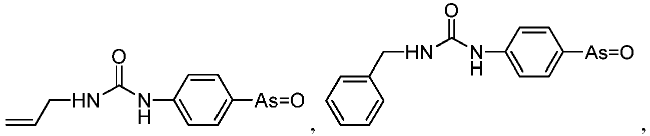

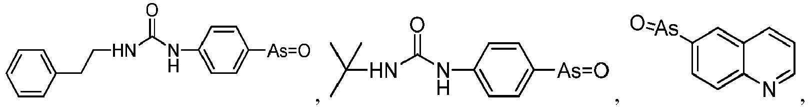

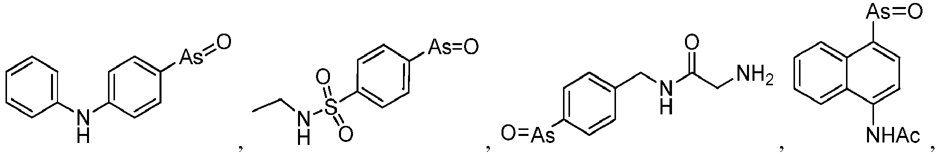

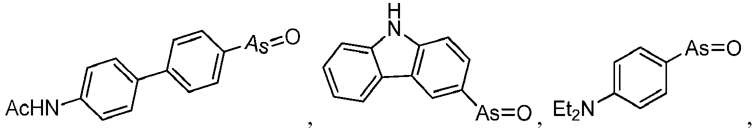

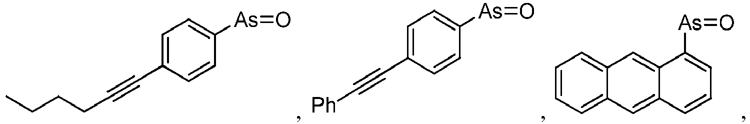

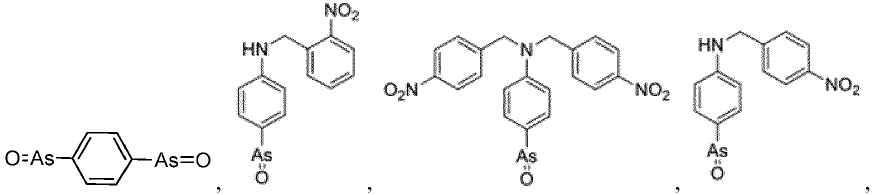

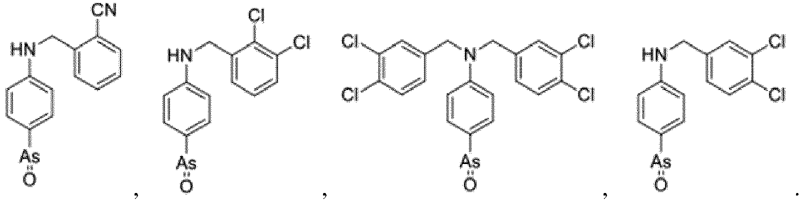

In some embodiments, the antibody is a monoclonal antibody or a polyclonal antibody.

In some embodiments, the antibody is a chimeric antibody, a humanized antibody, or a fully human antibody.

In some embodiments, the RNAi molecule is a small interfering RNA (siRNA), a short hairpin RNA (shRNA), or a microRNA (miRNA).

In some embodiments, the RNAi molecule has a length of 18-100 bases.

In some embodiments, the RNAi molecule is modified to enhance its stability.

In some embodiments, the subject is a human or a mammal.

In some embodiments, the mood disorder is an elevated mood, a depressed mood, or a cycle of elevated and depressed moods.

In some embodiments, the mood disorder is anxiety, depression, schizophrenia, or mania.

In some embodiments, the mood disorder is caused by a neurodegenerative disease, stroke, or malignant tumor.

In some embodiments, the mood disorder is anxiety or depression caused by a neurodegenerative disease.

In some embodiments, the PI4KIIIα/FAM126/TTC7 complex inhibitor is administered orally, subcutaneously, intramuscularly, or intravenously.

In some embodiments, the method further comprises diagnosing the subject as having the mood disorder before administering an effective amount of PI4KIIIα/FAM126/TTC7 complex inhibitor to the subject.

In some embodiments, the method further includes administering a second reagent to the subject in need thereof.

In some embodiments, the second reagent is used for treating the mood disorder.

In some embodiments, the second reagent is used for treating a neurodegenerative disease, stroke, or malignant tumor.

In some embodiments, the PI4KIIIα/FAM126/TTC7 complex inhibitor is administered before, after, or simultaneously with the second reagent.

According to another aspect, the present application provides use of a PI4KIIIα/FAM126/TTC7 complex inhibitor in manufacture of a medicament for preventing or treating a mood disorder.

According to still another aspect, the present application provides use of a PI4KIIIα/FAM126/TTC7 complex inhibitor in preventing or treating a mood disorder, including administering an effective amount of PI4KIIIα/FAM126/TTC7 complex inhibitor to a subject in need thereof.

### BRIEF DESCRIPTION OF THE DRAWINGS

FIG. 1 shows changes in weight of rats in each group during CUMS modeling and administration.
FIG. 2 shows that the mRNA expression level of PIK4IIIα is linearly positively correlated with the degree of depression.
FIG. 3 shows the PI4KIIIα inhibitor PAO can prevent or alleviate anxiety behavior in APP/PS1 mice (WT represents wild-type mice, "APP/PS1 0" represents APP/PS1 mice without treatment by PAO, "APP/PS1 0.3" represents mice with treatment by PAO, the dose is 0.3 mg/kg, and "^{∗}" represents P<0.05).
FIG. 4 shows the effect of PAO and Aricept on APP/PS 1 mice in the Morris water maze test.
FIG. 5 shows the effect of PAO and Aricept on APP/PS 1 mice in the Novelty suppressed feeding test.
FIG. 6 shows the effect of reducing the mRNA expression of Efr3a on APP/PS 1 mice in the light-dark transtition test.
FIG. 7 shows the effect of PAO derivatives PI04, PI05, and PI06 on ICR mice in the Forced swimming test. The black and gray bars are the sum of the immobile time within the last four minutes in the first and second forced swimming experiments for each group of mice respectively.
FIG. 8 shows the effect of PAO on the prevention or alleviation of depression in mice caused by neuronal damage.
FIG. 9 shows the effect of PAO and its derivatives on the inhibition of anxiety behavior in wild-type mice.

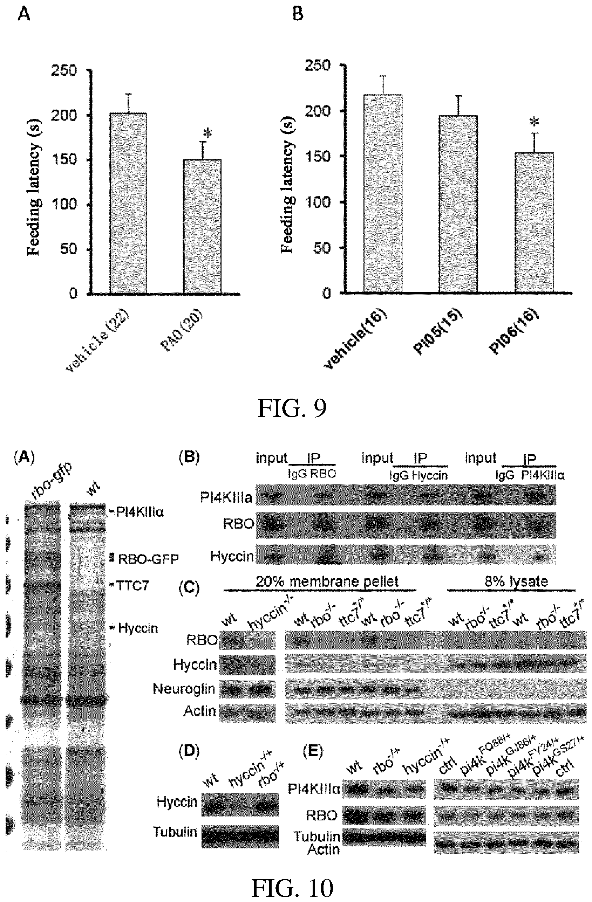

FIG. 10 shows the interaction of Hyccin (FAM126), RBO, TTC7, and P4KIIIα in Drosophila. FIG. 10A shows Coomassie brilliant blue staining of co-immunoprecipitates of the anti-GFP antibody from homogenates of adult Drosophila heads of *rbo-egfp* transgene and wild-type (wt) The expression of RBO-GFP is driven by the *rbo* gene driver. FIG. 10B shows that, by co-immunoprecipitation-western blotting (WB), the endogenous Hyccin, RBO, and PI4KIIIα (PI4K) co-immunoprecipitate with each other in the adult wt Drosophila. FIG. 10C shows, by WB, the expression levels of RBO and Hyccin in the cell membrane and cytoplasm of wt Drosophila embryos, and *hyccin*^{*-*/}*⁻, rbo*^{*-*/}*⁻,* and *ttc7*^{*∗*/*∗*} mutant Drosophila embryos. RBO is only expressed on the cell membrane and is not expressed in the embryos of the above three homozygous mutants. Hyccin is mainly present in the cytoplasm, and has the level with no significant change in the cytoplasm of the *rbo^{-l-}* and *ttc7*^{*∗*/*∗*} mutants, indicating that the expression of Hyccin is independent of the expression of RBO and TTC7. FIG. 10D shows, by western blotting (WB), the expression amount of Hyccin in the *rbo*^{*-*/*+*} and *hyccin^{-l+}* adult heterozygous mutant Drosophila, further indicating that the expression of Hyccin is independent of RBO. FIG. 10E: WB shows the total level of PI4IIIα and RBO in the *wt, rbo^{-l+},* and *hyccin^{-l+}* heterozygous mutant adult Drosophila with the same gene background, and in the *ctrl* and four nonsense mutation heterozygotes of *pi4kIIIa* (*pi4k*^{FQ88/+}*, pi4k*^{FY24/+}, *pi4k*^{GJ86/+}, and *pi4k*^{GS211+}) adult Drosophila. Wherein the four nonsense mutations of *pi4kIIIα* are obtained from the *ctrl* Drosophila by treatment with gene mutagen and screening.

### DETAILED DESCRIPTION

The following description of the present invention is intended only to describe various embodiments of the present invention. The specific embodiments described should not be construed to limit the scope of the present invention. Various equivalent substitutions, changes, or variations may be made by those skilled in the art without departing from the spirit and essence of the present invention, it is to be understood that these equivalent embodiments are also encompassed herein. All documents cited herein, including publications, patents, patent applications, etc., are incorporated by reference in their entirety.

According to an aspect, the present application provides a method for preventing or treating a mood disorder, including administering an effective amount of PI4KIIIα/FAM126/TTC7 complex inhibitor to a subject in need thereof.

### PI4KIIIα/FAM126/TTC7 complex inhibitor

In the present application, the term "PI4KIIIα/FAM126/TTC7 complex" refers to a complex composed of three proteins PI4KIIIα, FAM126, and TTC7, which localizes on the cytoplasmic membrane and participates in lipid phosphorylation in vivo. EFR3a or EFR3b is an important molecule that anchors the PI4KIIIα/FAM126/TTC7 complex to the cytoplasmic membrane.

In the present application, the term "PI4KIIIα/FAM126/TTC7 complex inhibitor" refers to any substance that can decrease, reduce, and eliminate the activity of the PI4KIIIα/FAM126/TTC7 complex. In some embodiments, the PI4KIIIα/FAM126/TTC7 complex inhibitor is an inhibitor that inhibits the transcription or translation of PI4KIIIα or FAM126 or TTC7 or EFR3 gene. In some other embodiments, the PI4KIIIα/FAM126/TTC7 complex inhibitor is an inhibitor that inhibits the capability of PI4KIIIα to phosphorylate lipids. In some other embodiments, the PI4KIIIα/FAM126/TTC7 complex inhibitor is an inhibitor that inhibits the formation of the PI4KIIIα/FAM126/TTC7 complex. In some other embodiments, the PI4KIIIα/FAM126/TTC7 complex inhibitor is an inhibitor that inhibits the PI4KIIIα/FAM126/TTC7 complex from being anchored to the cytoplasmic membrane.

In some embodiments, the PI4KIIIα/FAM126/TTC7 complex inhibitor can reduce the activity of the PI4KIIIα/FAM126/TTC7 complex by at least 5%, 10%, 20%, 40%, 50%, 80%, 90%, 95%, or more.

In the present application, the "activity", when used in conjunction with increase or decrease, means that the detected functional activity, which may be expressed as a changed content or a changed functional activity with an unchanged content. In some embodiments, the activity is associated with lipid phosphorylation. In some embodiments, the activity is associated with a mood disorder.

In some embodiments, the PI4KIIIα/FAM126/TTC7 complex inhibitor is a small-molecule compound, an antibody, an RNAi molecule, or an antisense nucleic acid.

In some embodiments, the PI4KIIIα/FAM126/TTC7 complex inhibitor is a small-molecule compound.

In the present application, the term "small-molecule compound" refers to a natural or chemically synthesized organic compound with a molecular weight less than 3000, 2500, 2000, 1500, 1000, or 500 daltons.

In some embodiments, the PI4KIIIα/FAM126/TTC7 complex inhibitor is an antibody.

In the present application, the term "antibody" includes any immunoglobulin, monoclonal antibody, polyclonal antibody, polyvalent antibody, bivalent antibody, monovalent antibody, or antibody that can bind to a specific antigen. In the present application, the term "antibody" is intended to broadly encompass conventional antibodies with four chains and unconventional antibodies without four chains (for example, antibodies that naturally lack light chains).

A conventional intact antibody is a heterotetramer containing two heavy (H) chains and two light (L) chains. There are five types of mammalian heavy chain: α, δ, ε, γ, and µ. Each heavy chain consists of one variable domain (V_{H}) and first, second, and third constant domains (C_{H1}, C_{H2}, and C_{H3}). There are two types of mammalian light chain: λ and κ. Each light chain consists of one variable domain (V_{L}) and one constant domain. Conventional antibodies have the "Y"-shaped structure with neck consisting of the second and third constant domains of two heavy chains joined by disulfide bonds. Each arm of the "Y"-shaped structure comprises the variable domain and the first constant domain of one of the two heavy chains that are joined with the variable domain and the constant domain of one light chain. The variable domains of the light chain and heavy chain determine the binding to an antigen. The variable domain of each chain contains three hypervariable regions referred to as complementarity-determining regions (CDRs) (CDRs of the light chain include LCDR1, LCDR2, and LCDR3, and CDRs of the heavy chain include HCDR1, HCDR2, and HCDR3). The CDR boundaries of the antibodies and antigen-binding fragments disclosed in the present application can be named or identified by Kabat, IMGT, Chothia, or Al-Lazikani nomenclature (Al-Lazikani, B., Chothia, C., Lesk, A. M., J. Mol. Biol., 273(4), 927 (1997); Chothia, C. et al., J Mol Biol. Dec 5;186(3):651-63 (1985); Chothia, C and Lesk, A.M., J.Mol.Biol., 196,901 (1987); Chothia, C. et al., Nature. Dec 21-28;342(6252):877-83 (1989); Kabat E.A. et al., Sequences of Proteins of Immunological Interest, 5th Ed. Public Health Service, National Institutes of Health, Maryland, Bethesda (1991); Marie-Paule Lefranc et al., Developmental and Comparative Immunology, 27: 55-77 (2003); Marie-Paule Lefranc et al., Immunome Research, 1(3), (2005); Marie-Paule Lefranc, Molecular Biology of B cells (second edition), chapter 26, 481-514, (2015)). Wherein three CDRs are separated by a lateral contiguous portion of a framework region (FR), and the FR is more highly conserved than the CDR and forms a scaffold to support the hypervariable loop. The constant region of the heavy chain and light chain is not involved in the binding to an antigen but has multiple effector functions.

Antibodies may be divided into several classes based on the amino acid sequence of the constant region of the heavy chain. The heavy chain types α, δ, ε, γ, and µ give rise to five main classes or isotypes of the antibody: IgA, IgD, IgE, IgG, and IgM. The main antibody classes may be further subdivided into subclasses such as IgG1 (γ1 heavy chain), IgG2 (γ2 heavy chain), IgG3 (γ3 heavy chain), IgG4 (γ4 heavy chain), IgA1 (α1 heavy chain), or IgA2 (α2 heavy chain), etc.

In some embodiments, the antibody is a full-length antibody or an antigen-binding fragment.

In the present application, the term "antigen-binding fragment" refers to an antibody fragment formed by an antibody fragment without an entire antibody structure but comprising one or more CDRs. For example, the antigen-binding fragment includes, but is not limited to, a Fab fragment, a Fab' fragment, a F(ab')₂ fragment, an Fv fragment, a single-chain variable fragment (scFv), an scFv dimer, a camelized single domain antibody, and a nanobody. The antigen-binding fragment can bind to the same antigen as the parent antibody.

The "Fab" fragment of an antibody refers to the portion of the antibody consisting of one light chain (including a variable domain and a constant domain) and a variable domain and a first constant domain of one heavy chain, which are jointed by disulfide bonds.

The "Fab‴ fragment refers to a Fab fragment that contains part of the hinge region.

The "F(ab')₂" fragment refers to a dimer of the Fab'.

The "Fv" fragment of an antibody consists of the variable domain of one light chain and the variable domain of one heavy chain.

The "single chain antibody molecule" or "scFv" refers to an engineered antibody made from the variable domain of a light chain and the variable domain of a heavy chain that are jointed directly or by a peptide chain. Details can be found in Huston JS et al., Proc Natl Acad Sci USA, 85:5879 (1988).

The "scFv dimer" refers to an oligomer formed by two scFvs.

The "camelized single domain antibody" (also referred to as "heavy-chain antibody" or "heavy-chain-only antibody (HCAb)") refers to an antibody that contains two heavy chain variable domains and no light chain (Riechmann L. and Muyldermans S., J Immunol Methods. Dec 10;231(1-2):25-38 (1999); Muyldermans S., J Biotechnol. Jun;74(4):277-302 (2001); WO94/04678; WO94/25591; U.S. Patent No. 6,005,079). The heavy-chain antibody is originally derived from the family Camelidae (camelids, dromedaries, and llamas). Although lack of the light chain, the camelized single domain antibody has all the functions for the binding to an antigen (referring to Hamers-Casterman C. et al., Nature. 363(6428):446-8 (1993); Nguyen VK. et al., "Heavy-chain antibodies in Camelidae; a case of evolutionary innovation," Immunogenetics. 54(1):39-47 (2002); Nguyen VK. et al., Immunology. 109(1):93-101 (2003)), which is incorporated by reference in its entirety.

The "nanobody" consists of one heavy chain variable domain from the heavy-chain antibody and two constant domains C_{H2} and C_{H3}.

In some embodiments, the antibody is a monoclonal antibody or a polyclonal antibody.

In some embodiments, the antibody is a murine antibody, a rabbit antibody, a chimeric antibody, a humanized antibody, or a fully human antibody.

In the present application, the term "fully human", when used in the antibody or antigen-binding fragment, means that the amino acid sequence of the antibody or antigen-binding fragment corresponds to the amino acid sequence of an antibody produced by a human or human immune cells or derived from non-human sources such as transgenic non-human animals based on the human antibody library, or other sequences encoding human antibodies.

In the present application, the term "humanized", when used in the antibody or antigen-binding fragment, refers to an antibody or antigen-binding fragment including CDRs derived from non-human animals, FRs derived from humans, and, where applicable, constant domains derived from humans. The humanized antibody or antigen-binding fragment may be used in some embodiments as a therapeutic agent for humans since it is less immunogenic. In some embodiments, the non-human animals are mammals (such as mice, rats, rabbits, goats, sheep, guinea pigs or hamsters). In some embodiments, the humanized antibody or antigen-binding fragment consists essentially entirely of human sequences, except that the CDR sequences are non-human.

In the present application, the term "chimeric", when used in the antibody or antigen-binding fragment, refers to an antibody or antigen-binding fragment including a heavy chain and/or a light chain with one portion derived from the same species and the other portions derived from different species. In some embodiments, the chimeric antibody may include a constant domain derived from human and a variable domain derived from a non-human animal (such as a mouse or a rabbit).

In some embodiments, the antibody in the present application is a monospecific antibody, a bispecific antibody, or a multispecific antibody.

In some embodiments, the antibody in the present application may be further marked.

In some embodiments, the antibody in the present application is an antibody specific to PI4KIIIα or FAM126 or TTC7 or EFR3. In some embodiments, the antibody in the present application is an antibody specific to PI4KIIIα or EFR3.

In some embodiments, the antibody in the present application can inhibit the formation of the PI4KIIIα/FAM126/TTC7 complex, inhibit the PI4KIIIα/FAM126/TTC7 complex from being anchored to the cytoplasmic membrane, or inhibit the phosphorylation activity of the PI4KIIIα/FAM126/TTC7 complex.

In some embodiments, the PI4KIIIα/FAM126/TTC7 complex inhibitor is an RNAi molecule.

In the present application, the term "RNAi molecule" refers to an RNA or analog thereof that is sufficiently complementary in sequence to a target RNA to direct RNA interference. In some embodiments, DNA that can be used to produce RNA is also involved. RNAi is a process of sequence-specific selection in which target molecules (such as target gene, protein, or RNA) are downregulated.

In some embodiments, the RNAi molecule is a small interfering RNA (siRNA), a short hairpin RNA (shRNA), or a microRNA (miRNA).

In the present application, the term "small interfering RNA (siRNA)" refers to an RNA molecule, preferably a double-stranded molecule, with a length of about 10-50 nucleotides, preferably a length of about 15-25 nucleotides, more preferably a length of about 17, 18, 19, 20, 21, 22, 23, 24, or 25 nucleotides. Optionally, the strand has an overhanging end including, for example, one, two, or three overhanging nucleotides (or nucleotide analogs) that can direct or mediate the degradation of RNA. Naturally occurring siRNAs are produced from longer dsRNAs (with >25 nucleotides) through the RNAi machinery (such as Dicer or a homologue thereof) of cells, and form the RISC complex to degrade mRNA.

In the present application, the term "short hairpin RNA (shRNA)" refers to an RNA molecule with a stem-loop structure that includes a first region and a second region that are complementary to each other. The degree of complementarity and the orientation of the regions are sufficient for base pairs to occur between the regions. The first region and the second region are linked by a loop region. The loop results from the absence of base pairing between nucleotides (or nucleotide analogs) in a loop region.

In the present application, the term "microRNA" or "miRNA" is a short and naturally occurring single-stranded non-coding RNA molecule with a length of about 16-26 nucleotides (nt) (for example, about 16-29 nt, 19-22 nt, 20-25 nt, or 21-23 nt), which usually functions in regulation of gene expression in vivo. In eukaryotic cells, a miRNA gene is transcribed by DNA transcriptase II into a "primary product" (pri-miRNA), the pri-miRNA is quickly processed by a ribonuclease III (Drosha) into a miRNA "precursor" (pre-miRNA), the pre-miRNA is transported from the nucleus to the cytoplasm, and then recognized and cleaved by another ribonuclease III (Dicer) into a mature miRNA. The mature miRNA molecule is partially complementary to one or more mRNAs and regulates expression of protein. Known miRNA sequences can be obtained from public databases, for example, the miRBase database (www.mirbase.org), providing miRNA sequence information, functional annotations, predicted gene targets, and other information. In the present invention, miRNA also includes RNA molecules with similar structure and function to natural miRNAs that are expressed in cells by artificially synthesized plasmids, and target corresponding mRNAs like natural miRNA molecules to prevent the translation of the mRNAs into proteins.

In some embodiments, the RNAi molecule can reduce the expression of PI4KIIIα, for example, by knocking down the PI4KA gene.

In some embodiments, the RNAi molecule has a sequence of SEQ ID NO: 1 [5'-TGCTCATTAGCAGTAAAGA-3'].

In some embodiments, the RNAi molecule has a length of 18-100 bases.

In some embodiments, the RNAi molecule is modified to enhance its stability.

In some embodiments, the PI4KIIIα/FAM126/TTC7 complex inhibitor is an antisense nucleic acid.

In the present application, the term "antisense nucleic acid" includes nucleotides that are fully complementary to a target sequence and nucleotides with one or more nucleotide mismatches, so long as the antisense nucleic acid can specifically hybridize to the target sequence. For example, the antisense nucleic acid in the present application includes polynucleotides with at least 70% or more, preferably 80% or more, more preferably 90% or more, even more preferably 95% or more homology in a length of at least 15 contiguous nucleotides. Transcription of the target gene and/or translation of the target mRNA are/is reduced or blocked due to the formation of a hybrid. The standard method involving antisense technology is described (see, for example, Melani et al., Cancer Res.(1991)51:2897-2901).

In some embodiments, the PI4KIIIα/FAM126/TTC7 complex inhibitor is an EFR3-specific inhibitor.

In the present application, the term "EFR3-specific inhibitor" refers to any inhibitor that can specifically decrease, reduce, and eliminate the transcription or translation of the EFR3 (i.e., yeast-derived formyl-CoA reductase) gene and/or the activity of the EFR3 protein. In some embodiments, the EFR3-specific inhibitor can reduce the activity of EFR3 by at least 5%, 10%, 20%, 40%, 50%, 80%, 90%, 95%, or more. In some embodiments, the activity of EFR3 refers to the activity of anchoring the PI4KIIIα/FAM126/TTC7 complex to the cytoplasmic membrane.

In some embodiments, the EFR3-specific inhibitor can preferably recognize the EFR3 protein from a complex mixture. A binding constant of the inhibitor to the EFR3 protein is at least two times a binding constant of the inhibitor to another non-specific binding protein. In some embodiments, the equilibrium dissociation constant of the EFR3-specific inhibitor from the EFR3 protein is less than or equal to 10⁻⁵ M or 10⁻⁶ M. In some embodiments, the equilibrium dissociation constant of the EFR3-specific inhibitor from the EFR3 protein is less than or equal to 10⁻⁶ M or 10⁻⁷ M. In some embodiments, the equilibrium dissociation constant of the EFR3-specific inhibitor from the EFR3 protein is less than or equal to 10⁻⁷ M or 10⁻⁸ M. In some embodiments, the EFR3-specific inhibitor is an EFR3a-specific inhibitor or an EFR3b-specific inhibitor.

In some embodiments, the PI4KIIIα/FAM126/TTC7 complex inhibitor is a PI4KIIIα-specific inhibitor.

In the present application, the term "PI4KIIIα-specific inhibitor" refers to any substance that can specifically decrease, reduce, and eliminate the transcription or translation of the PI4KIIIα gene and/or the activity of the PI4KIIIα protein. In some embodiments, the PI4KIIIα-specific inhibitor can reduce the activity of PI4KIIIα by at least 5%, 10%, 20%, 40%, 50%, 80%, 90%, 95%, or more. In some embodiments, the activity of PI4KIIIα refers to the activity of PI4KIIIα to phosphorylate (for example, the conversion into phosphatidylinositol-4-phosphate (PI4P)) lipids (for example, phosphoinositide (PI)).

In some embodiments, the PI4KIIIα-specific inhibitor can preferably recognize the PI4KIIIα protein from a complex mixture. A binding constant of the inhibitor to the PI4KIIIα protein is at least two times a binding constant of the inhibitor to another non-specific binding protein. In some embodiments, the equilibrium dissociation constant of the PI4KIIIα-specific inhibitor from the PI4KIIIα protein is less than or equal to 10⁻⁵ M or 10⁻⁶ M. In some embodiments, the equilibrium dissociation constant of the PI4KIIIα-specific inhibitor from the PI4KIIIα protein is less than or equal to 10⁻⁶ M or 10⁻⁷ M. In some embodiments, the equilibrium dissociation constant of the PI4KIIIα-specific inhibitor from the PI4KIIIα protein is less than or equal to 10⁻⁷ M or 10⁻⁸ M.

In some embodiments, the PI4KIIIα-specific inhibitor inhibits PI4KIIIα at least 1-fold, 2-fold, 4-fold, 5-fold, 10-fold, 20-fold, 30-fold, 50-fold, 100-fold, 200-fold, 500-fold, or 10000-fold more than to inhibit PI4KII (such as PI4KIIα or PI4KIIβ). In some embodiments, the PI4KIIIα-specific inhibitor inhibits PI4KIIIα at least 1-fold, 2-fold, 4-fold, 5-fold, 10-fold, 20-fold, 30-fold, 50-fold, 100-fold, 200-fold, 500-fold, or 10000-fold more than to inhibit PI4KIIIβ. In some embodiments, the PI4KIIIα-specific inhibitor substantially does not inhibit PI4KII (such as PI4KIIα or PI4KIIβ), with IC₅₀ greater than or equal to 10 µM, 20 µM, 30 µM, 40 µM, 50 µM, 60 µM, 80 µM, 100 µM, 150 µM, 200 µM, or 500 µM.

In some embodiments, the PI4KIIIα-specific inhibitor inhibits PI4KIIIα with IC₅₀ less than or equal to 100 µM, 80 µM, 50 µM, 30 µM, 20 µM, 10 µM, 5 µM, 3 µM, 2 µM, 1 µM, 0.5 µM, 0.2 µM, 0.1 µM, 0.05 µM, 0.02 µM, 0.01 µM, 0.005 µM, 0.002 µM, or 0.001 µM.

In some embodiments, the PI4KIIIα-specific inhibitor is a small-molecule compound.

In some embodiments, the small-molecule compound in the present application has a structure of formula (I) or a pharmaceutically acceptable salt thereof, 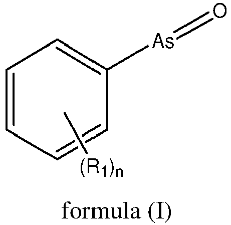 wherein R₁ is independently selected from (a) H, deuterium, halogen, nitro, cyano, hydroxyl, amino, carbamoyl, C₁₋₆ alkyl, C₂₋₆ alkynyl, C₂₋₆ alkenyl, C₁₋₆ alkoxy, C₁₋₆ haloalkyl, C₁₋₆ alkylene-NH₂, C₁₋₆ alkylene-NH-C(O)H, -As(O), -N=NH, -NH-(C₁₋₆ alkyl), - N,N-(C₁₋₆ alkyl)₂, -NH-C(O)H, -NH-S(O)₂H, -C(O)OH, -OC(O)H, -SH, -S(O)₂H, -S(O)₂-NH, or heterocyclyl, which is optionally substituted by R₂ or R₃, wherein R₂ and R₃ are each independently selected from amino, C₁₋₆ alkyl, C₁₋₆ alkoxy, C₁₋₆ haloalkyl, -NH-(C₁₋₆ alkyl), - NH-(6- to 12-membered aryl), -N,N-(C₁₋₆ alkyl)₂, C₃₋₆ cycloalkyl, 6- to 12-membered aryl, or 3- to 12-membered heterocyclyl, which is optionally substituted by one or more of halogen, nitro, cyano, hydroxyl, amino, carbamoyl, -NH-C(O)-R₅, -C(O)OR₄, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-, R₄ is C₁₋₆ alkyl, which is optionally substituted by one or more of halogen, nitro, cyano, hydroxyl, amino, carbamoyl, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-, and R₅ is selected from H, C₁₋₆ alkyl, C₂₋₆ alkynyl, C₂₋₆ alkenyl, C₁₋₆ alkoxy, or C₁₋₆ haloalkyl, or
(b) R₁ on two adjacent carbon atoms forms 5- to 12-membered cycloalkyl, aryl, or heterocyclyl, which is optionally substituted by one or more of halogen, nitro, cyano, hydroxyl, amino, carbamoyl, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-,

In some embodiments, n is 0, 1, 2, or 3. In some embodiments, n is 0, 1, or 2. In some embodiments, n is 0 or 1.

In some embodiments, R₁ is independently selected from H, deuterium, halogen, nitro, cyano, hydroxyl, amino, carbamoyl, C₁₋₆ alkyl, C₁₋₆ alkoxy, C₁₋₆ haloalkyl, - As(O), -NH-(C₁₋₆ alkyl), -N,N-(C₁₋₆ alkyl)₂, or -C(O)OR₆, wherein n is an integer from 0 to 2, and R₆ is C₁₋₆ alkyl.

In some embodiments, R₁ is independently selected from H, deuterium, halogen, nitro, cyano, hydroxyl, amino, C₁₋₆ alkyl, C₁₋₆ alkoxy, C₁₋₆ haloalkyl, or -As(O), wherein n is an integer from 0 to 2.

In some embodiments, R₁ is independently selected from H, deuterium, halogen, amino, C₁₋₆ alkoxy, or C₁₋₆ haloalkyl, wherein n is 1.

In some embodiments, R₁ is located at an ortho position or para position of the -As(O) group. In some embodiments, R₁ is H.

In the present application, the term "substituted" means that one or more hydrogen atoms of the chemical group are removed and substituted with a substituent.

In the present application, the term "substituent", having its common meaning known in the art, refers to a chemical moiety that is covalently linked or, where appropriate, fused to a parent group.

In the present application, the term "Cₙ-Cₘ" represents a range of carbon atom numbers, wherein n and m are integers and the range of carbon numbers includes the endpoints (i.e., n and m) and integer points therebetween. For example, C₁-C₆ represents a range of 1 to 6 carbon atoms, including 1, 2, 3, 4, 5, and 6 carbon atoms.

In the present application, the term "alkyl", whether used as part of another term or alone, refers to a saturated hydrocarbyl group that may be straight-chained or branched-chained. The term "Cₙ-Cₘ alkyl" refers to an alkyl group with n to m carbon atoms. In some embodiments, the alkyl group contains 1 to 12, 1 to 8, 1 to 6, 1 to 4, 1 to 3, or 1 to 2 carbon atoms. For example, the alkyl group includes, but is not limited to, chemical groups such as methyl, ethyl, n-propyl, isopropyl, n-butyl, tert-butyl, isobutyl, sec-butyl, 2-methyl-1-butyl, n-pentyl, 3-pentyl, n-hexyl, and 1,2,2-trimethylpropyl, etc.

In the present application, the term "alkenyl", whether used as part of another term or alone, refers to an unsaturated hydrocarbyl group that may be straight-chained or branched-chained, with at least one carbon-carbon double bond. In some embodiments, the alkenyl group contains 2 to 12, 2 to 10, 2 to 8, 2 to 6, 2 to 5, 2 to 4, or 2 to 3 carbon atoms. In some embodiments, the alkenyl group may also contain 1 to 6, 1 to 5, 1 to 4, 1 to 3, 1 to 2, or 1 carbon-carbon double bond. For example, the alkenyl group includes, but is not limited to, chemical groups such as vinyl, n-propenyl, isopropenyl, n-butenyl, and sec-butenyl, etc.

In the present application, the term "alkynyl", whether used as part of another term or alone, refers to an unsaturated alkynyl group that may be straight-chained or branched-chained, with at least one carbon-carbon triple bond. In some embodiments, the alkynyl group contains 2 to 12, 2 to 10, 2 to 8, 2 to 6, 2 to 5, 2 to 4, or 2 to 3 carbon atoms. In some embodiments, the alkynyl group may also contain 1 to 6, 1 to 5, 1 to 4, 1 to 3, 1 to 2, or 1 carbon-carbon triple bond. For example, the alkynyl group includes, but is not limited to, chemical groups such as ethynyl, propynyl, and butynyl, etc.

In the present application, the term "cycloalkyl" refers to a ring alkyl group consisting of at least 3 atoms. The term "n- to m- membered cycloalkyl" refers to a cycloalkyl group with n to m members for the formation of the ring. In addition, the ring may also contain one or more double bonds without a fully conjugated system. In some embodiments, the cycloalkyl group contains 3 to 8, 3 to 6, or 4 to 6 carbon atoms for the formation of the ring. For example, the cycloalkyl group includes, but is not limited to, cyclopropane, cyclobutane, and cyclopentyl, etc.

In the present application, the term "heterocyclyl" refers to a ring group in which at least one atom in the ring is a heteroatom and the remaining atoms are carbon atoms. The term "n- to m- membered heterocyclyl" refers to a heterocyclyl group with n to m members for the formation of the ring. In the present application, the term "heterocyclyl" includes heteroaryl and heterocycloalkyl. In addition, the ring may also contain one or more double bonds. In some embodiments, the heterocyclyl is a saturated heterocycloalkyl group. For example, the heteroatom includes, but is not limited to, oxygen, sulfur, nitrogen, and phosphorus, etc.

In the present application, the term "heterocycloalkyl" refers to a cycloalkyl group in which at least one atom in the ring is a heteroatom and the remaining atoms are carbon atoms. The term "n- to m- membered heterocycloalkyl" refers to a heterocycloalkyl group with n to m members for the formation of the ring. In addition, the ring may also contain one or more double bonds without a fully conjugated system. In some embodiments, the heterocycloalkyl is a saturated heterocycloalkyl group. For example, the heteroatom includes, but is not limited to, oxygen, sulfur, nitrogen, and phosphorus. In some embodiments, the heterocycloalkyl group contains 3 to 8, 3 to 6, or 4 to 6 carbon atoms for the formation of the ring. For example, the heterocycloalkyl group includes, but is not limited to, azetidine, aziridine, pyrrolidinyl, piperidinyl, piperazinyl, morpholinyl, thiomorpholine, and homopiperazine, etc.

In the present application, the term "aryl", whether used as part of another term or alone, refers to a mono- or poly-carbocyclic group with alternating double and single bonds between the carbon atoms forming the ring. The term "Cₙ-Cₘ aryl" refers to an aryl group with n to m carbon atoms for the formation of the ring. In some embodiments, the aryl ring system contains 6 to 12, 6 to 10, or 6 to 8 carbon atoms in one or more rings. In some embodiments, the aryl ring system contains two or more fused rings. For example, the aryl group includes, but is not limited to, chemical groups such as phenyl, naphthyl, tetrahydronaphthyl, indanyl, and indenyl, etc.

In the present application, the term "heteroaryl" refers to an aryl group in which at least one ring atom is a heteroatom and the remaining ring atoms are carbon atoms. The term "n- to m- membered heteroaryl" refers to a heteroaryl group with n to m members for the formation of the ring. For example, the heteroatom includes, but is not limited to, oxygen, sulfur, nitrogen, and phosphorus, etc. In some embodiments, the heteroaryl group may contain 5 to 10, 5 to 8, or 5 to 6 members for the formation of the ring. In some embodiments, the heteroaryl group is 5- or 6- membered heteroaryl. For example, the heteroaryl group includes, but is not limited to, furyl, thienyl, pyridyl, quinolyl, pyrrolyl, N-lower alkylpyrrolyl, pyridyl-N-oxide, pyrimidinyl, pyrazinyl, imidazolyl, and indolyl.

In the present application, the term "alkoxy", whether used as part of another term or alone, refers to the "-O-alkyl" group. The term "Cₙ-Cₘ alkoxy" means that the alkyl portion of the alkoxy group contains n to m carbon atoms. In some embodiments, the alkyl portion contains 1 to 6, 1 to 4, or 1 to 3 carbon atoms. For example, the alkoxy group includes, but is not limited to, chemical groups such as methoxy, ethoxy, propoxy (e.g., n-propoxy and isopropoxy), and t-butoxy, etc.

In the present application, the term "haloalkyl", whether used as part of another term or alone, refers to the "-alkyl-X" group, wherein X is a halogen selected from the group consisting of fluorine, chlorine, bromine and iodine. The term "Cₙ-Cₘ haloalkyl" means that the alkyl portion of the haloalkyl group contains n to m carbon atoms. In some embodiments, the alkyl portion contains 1 to 6, 1 to 4, or 1 to 3 carbon atoms. For example, the haloalkyl group includes, but is not limited to, chemical groups such as halomethyl, haloethyl, halopropyl (e.g., n-halopropyl and isohalopropyl), and t-halobutyl, etc.

In the present application, the term "n-membered", wherein n is an integer, is generally used with a ring system to describe the number of atoms in the ring system that form the ring. For example, piperidinyl is one of 6-membered heterocycloalkyl groups, pyrazolyl is one of 5-membered heteroaryl groups, pyridyl is one of 6-membered heteroaryl groups, and 1,2,3,4-tetrahydro-naphthalene is one of 10-membered aryl groups.

In the present application, the term "halogen" refers to an atom selected from the group consisting of fluorine, chlorine, bromine and iodine.

In the present application, the term "cyano" refers to the "-CN" group.

In the present application, the term "hydroxyl" refers to the "-OH" group.

In the present application, the term "nitro" refers to the "-NO₂" group.

In the present application, the term "amino" refers to the "-NH₂" group.

In the present application, the term "carbamoyl" refers to the "-HNCONH₂" group.

In the present application, the term "compound" is intended to include all stereoisomers (such as enantiomers and diastereomers), geometric isomers, tautomers, and isotopes of the shown structure.

In the present application, the compound in the present application may be asymmetric (for example, with one or more stereocenters). Unless otherwise specified, all stereoisomers such as enantiomers and diastereomers are involved. The compound in the present application containing asymmetrically substituted carbon atoms may be isolated in an optically active or racemic form. Methods for preparing an optically active form from an initial raw material without optical activity are known in the art, such as resolution of a racemic mixture or stereoselective synthesis. The compound in the present application may also include various geometric isomers such as olefins and carbon-carbon double bonds, and all of these stable isomers have been encompassed in the present application. Cis and trans geometric isomers of the compound described in the present application may be isolated as a mixture of the isomers or as individual isomers.

In some embodiments, the compound in the present application has the *R* configuration. In some embodiments, the compound in the present application has the S configuration.

The racemic mixture of the compound may be resolved by any method known in the art. For example, the methods include fractional crystallization with a chiral resolving acid, a salt-forming organic acid with optical activity. Proper resolving reagents for the fractional crystallization are, for example, optically active acids (such as D and L forms of tartaric acid, diacetyltartaric acid, dibenzoyltartaric acid, mandelic acid, malic acid, and lactic acid) or various optically active camphorsulfonic acids. Other resolving reagents for the fractional crystallization include stereoisomerically pure forms such as N-methylbenzylamine, 2-phenylglycinol, norephedrine, ephedrine, N-methylephedrine, cyclohexylethylamine, and 1,2-diaminocyclohexane.

The resolution of the racemic mixture may be carried out by elution on a chromatography column with an optically active resolving reagents (for example, dinitrobenzoylphenylglycine). A proper solvent for elution may be determined by a person skilled in the art.

The compound in the present application also includes forms of tautomerism. The forms of tautomerism result from the exchange of a single bond with an adjacent double bond accompanied by the migration of protons. The forms of tautomerism include tautomers with protons in isomeric protonation states the same chemical formula and total charge. For example, the tautomers with protons include a keto-enol pair, an amide-imidic acid pair, a lactam-lactim pair, an enamine-imine pair, and a cyclic form, wherein the proton can occupy two or more positions of a heterocyclic system, for example, 1H- and 3H-imidazole; 1H-, 2H- and 4H-1,2,4-triazole; 1H- and 2H-isoindole; and 1H- and 2H-pyrazole. The forms of tautomerism can be equilibrated or sterically locked into one form by suitable substitution.

The compound in the present application may also include all isotopes of atoms occurring in the intermediates or the final compound. The isotopes include atoms that have the same atomic number but different mass number. For example, the isotopes of hydrogen include protium, deuterium, and tritium.

In some embodiments, the small-molecule compound in the present application may be organically synthesized. Any known organic synthesis technology and various possible synthetic routes may be used to prepare the compound in the present application including salts, esters, hydrates, or solvents thereof.

The compound in the present application may be prepared with a proper solvent that can be easily selected by a person skilled in the art of organic synthesis. The proper solvent is substantially unreactive with an initial raw material (reactant), an intermediate, or a product at the reaction temperature (for example, a temperature range from a freezing temperature of the solvent to a boiling temperature of the solvent). A given reaction can be carried out in one solvent or a mixture of more than one solvent. A person skilled in the art can select a proper solvent for a specific reaction step.

The preparation of the compound in the present application may involve the protection and deprotection of various chemical groups. A person skilled in the art can easily determine whether there is a need for protection or deprotection, and selection of a proper protection group. For the protection group in chemistry, refer to T. W. Greene and P. G. M. Wuts, Protective Groups in Organic Synthesis, 3rd Ed., Wiley & Sons, Inc., New York (1999), which is incorporated by reference in its entirety.

The reaction may be monitored according to any suitable method known in the art. For example, the formation of a product may be monitored by spectroscopy such as nuclear magnetic resonance spectroscopy (e.g., ¹H or ¹³C), infrared spectroscopy, spectrophotometry (e.g., UV-Vis), or mass spectrometry, or by chromatography such as high-performance liquid chromatography (HPLC), liquid chromatography-mass spectrometry (LCMS), or thin-layer chromatography (TLC). A person skilled in the art may purify the compound by various methods including high performance liquid chromatography (HPLC) (referring to "Preparative LC-MS Purification: Improved Compound Specific Method Optimization" Karl F. Blom, Brian Glass, Richard Sparks, Andrew P. Combs J. Combi. Chem. 2004, 6(6), 874-883, which is incorporated by reference in its entirety) and normal phase silica gel column chromatography.

In some embodiments, the small-molecule compound in the present application may be commercially available.

In some embodiments, the small-molecule compound is:

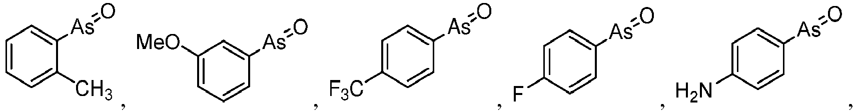

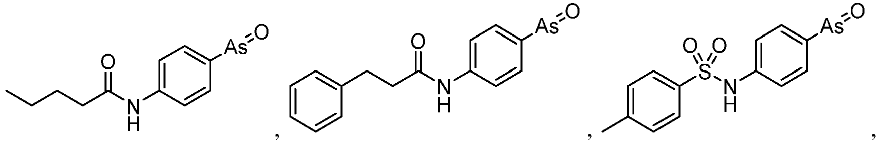

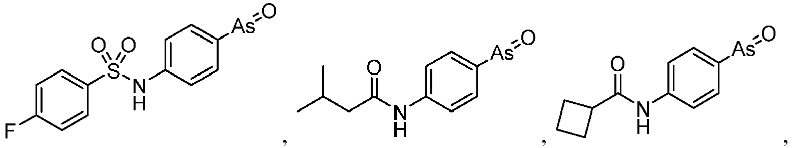

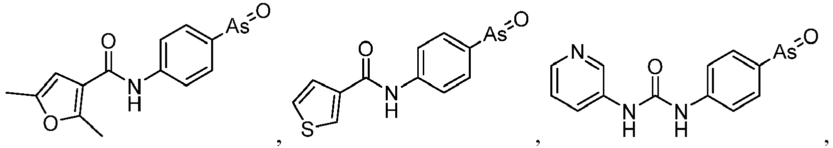

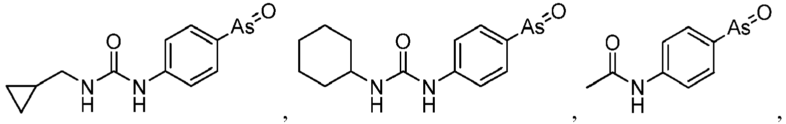

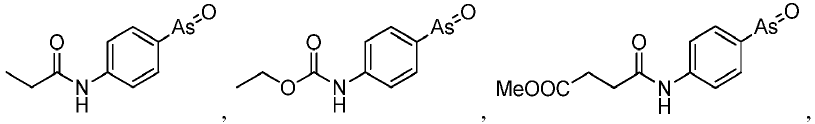

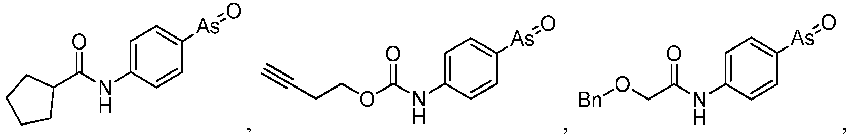

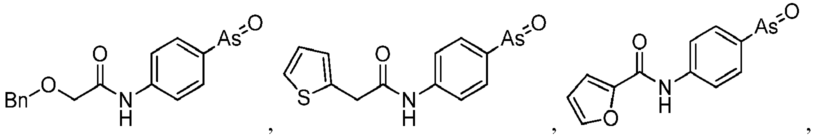

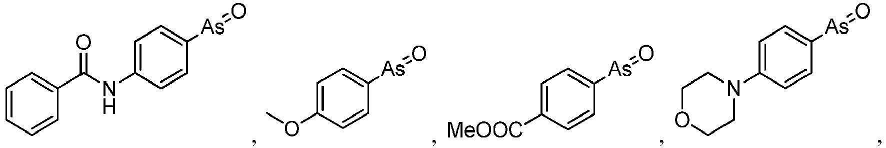

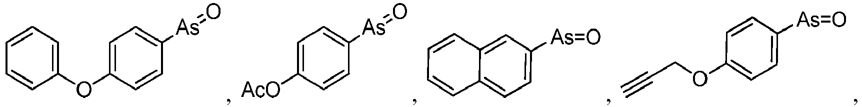

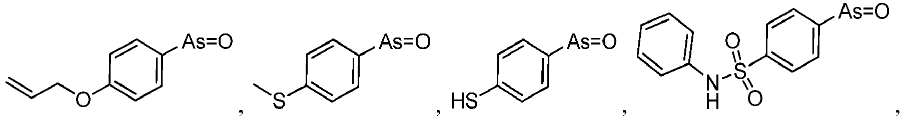

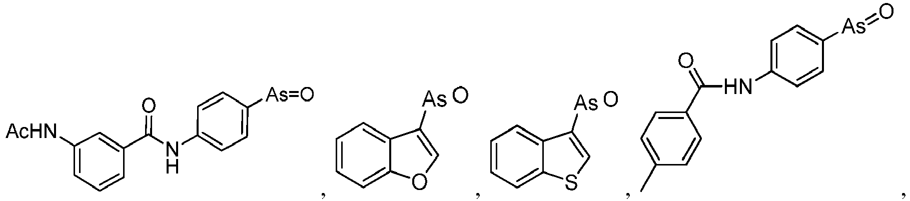

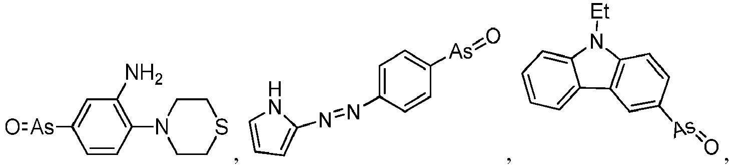

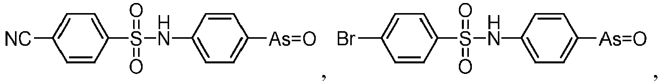

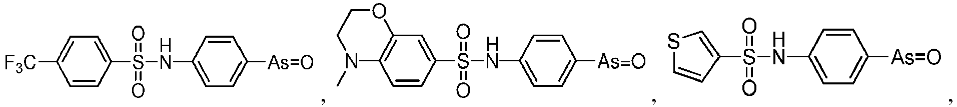

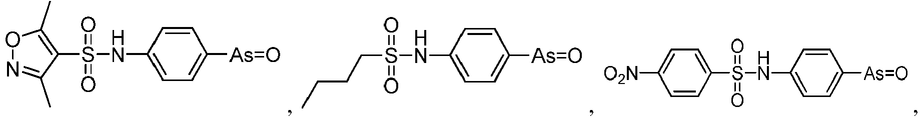

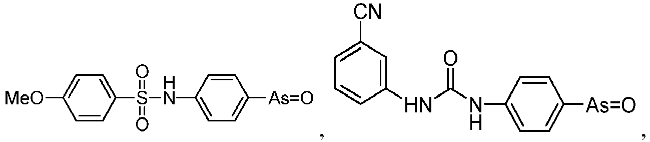

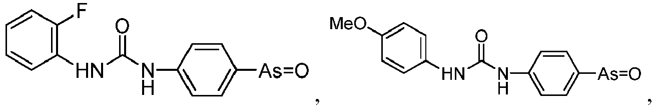

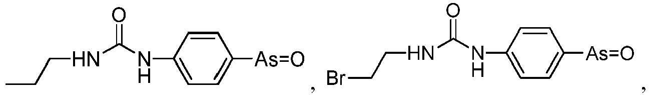

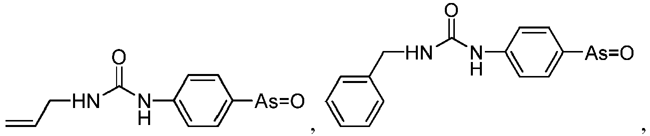

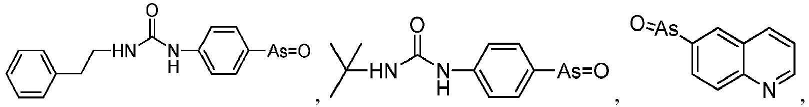

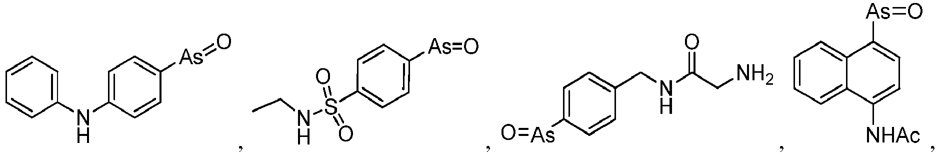

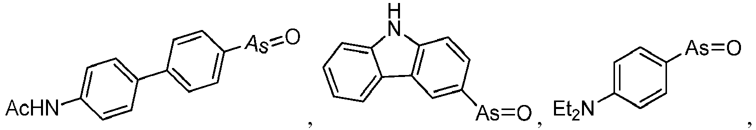

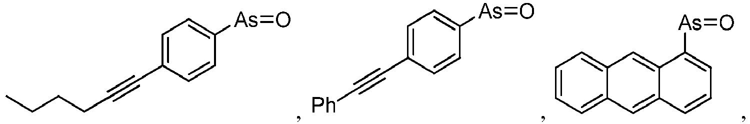

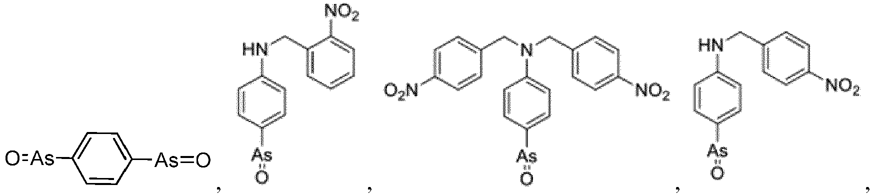

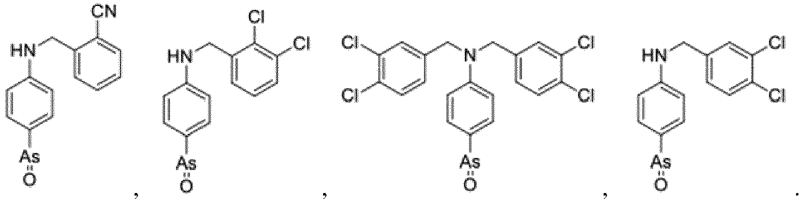

In the present application, the term "PAO" refers to a small-molecule compound with an arsenic oxide group and a benzene ring as the basic structure specifically as follows:

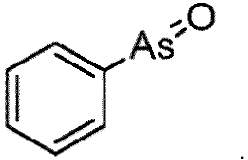

### Mood disorder

In the present application, the term "mood disorder" refers to a condition of distress due to an abnormal mood (affection) that persists for a certain period of time, causing some kind of disturbance to daily life.

In some embodiments, the mood disorder is an elevated mood, a depressed mood, or a cycle of elevated and depressed moods.

In the present application, the term "elevated mood" refers to the main primary symptom of a manic episode that typically manifests in feeling good by the patient, thus appearing elated, triumphant, carefree, and the like. This high-spirited mood is very infectious, often leading to laughter from people around. Some patients with manic episodes have the main manifestation of irritability, and they will lose their temper at every turn, and may even have destructive and aggressive behaviors.

In the present application, the term "depressed mood" refers to a mental state with feelings including, but not limited to, sadness and depression, leading to destructive behavior. The patient feels depressed, distressed, and sad, in low and gloomy tone. The depressed mood of a depression episode has two significant features: first, "significant and persistent", which means that this depressed mood is usually felt obviously by oneself and/or others from sullen to heartbroken, lasting for at least two weeks; and second, "not commensurate with actual situation", which means that this depressed mood is a distorted and magnified "depression", and is difficult to be explained with an objective reality.

In the present application, the term "cycle of elevated and depressed moods" refers to a mental state that alternates between an elevated mood and a depressed mood.

In some embodiments, the mood disorder is anxiety, depression, schizophrenia, or mania.

In the present application, the term "anxiety" includes panic disorder with or without agoraphobia, agoraphobia without a history of panic disorder, specific phobia, social anxiety disorder, obsessive-compulsive disorder, post-traumatic stress disorder, acute stress disorder, generalized anxiety disorder, anxiety disorders attributable to general medical conditions, substance-induced anxiety disorders, and anxiety disorders not otherwise listed. As used herein, the term "anxiety" includes those related disorders described in the diagnostic and statistical manual of anxiety (for example, referring to Diagnostic and Statistical Manual of Mental Disorders, DSM-IV, 1994, American Psychiatric Association, Washington, D.C.). A person skilled in the art should be aware that neurological and psychiatric disorders, particularly anxiety disorders, involve an alternative nomenclature system, a disease classification system, and a classification system that evolve with the development of medical science. Therefore, the term "anxiety" is intended to include similar disorders described in other diagnostic sources.

In the present application, the term "depression" includes, for example, single or recurrent major depressive and dysthymic disorders, depressive neuropathies, and functional depression. The depression in the present application includes: melancholic depression, including anorexia, excessive weight loss, insomnia and early-morning waking, and psychomotor retardation; atypical depression (or reactive depression), including increased appetite, hypersomnia, psychomotor agitation or irritability, anxiety disorders, and phobias; and seasonal affective disorder. As used herein, the term "depression" includes related disorders described in the DSM-IV.

In the present application, the term "schizophrenia" is a mental disorder characterized by severe chronic trauma, with the symptoms that appear in young adulthood, followed by a period of social impairment. The schizophrenia manifests as auditory and visual hallucinations, paranoia, delusions (positive symptoms), emotional blunting, depression, anhedonia, alogia, memory and attention deficits, social withdrawal (negative symptoms), and the like.

In the present application, the term "mania" is a psychiatric disorder mainly characterized by elevated emotions or irritability, accompanied by increased energy, increased speech, hyperactivity, and in severe cases, hallucinations, delusions, and stress. A mania episode lasts at least one week, usually in an episodic course. The patient goes into intermittent remission with normal mental status after each episode. Most patients tend to have recurrent episodes.

In some embodiments, the mood disorder is caused by a neurodegenerative disease, stroke, or malignant tumor.

In some embodiments, the mood disorder is anxiety or depression caused by a neurodegenerative disease, for example, anxiety or depression caused by Alzheimer's disease.

### Drug administration and medicinal use

In the present application, the term "pharmaceutically acceptable" refers to, within the scope of sound medical judgment, those compounds, materials, compositions, and/or dosage forms suitable for use in contact with human and animal tissues without undue toxicity, irritation, allergic reaction, or other problems or complications, with a proper return/risk ratio. In some embodiments, pharmaceutically acceptable compounds, materials, compositions, and/or dosage forms refer to those approved by a regulatory agency (such as the United States Food and Drug Administration, the China Food and Drug Administration, or the European Medicines Agency), or those listed in generally recognized pharmacopoeia (such as the United States Pharmacopoeia, the Chinese Pharmacopoeia, or the European Pharmacopoeia) for use in animals (more particularly, in humans).

In the present application, the term "subject" may include human and non-human animals. The non-human animals include all vertebrates such as mammals and non-mammals. The "subject" may be a livestock (such as cow, pig, sheep, chicken, rabbit, or horse), or a rodent (such as rat or mouse), or a primate (such as gorilla or monkey), or a domesticated animal (such as dog or cat). The "subject" may be male or female, and may also be of different ages. The human "subject" may be Caucasian, African, Asian, Semite, or other races, or a hybrid of different races. The human "subject" may be an elderly, adult, adolescent, child, or infant.

In some embodiments, the subject in the present application is a human or a mammal. In some embodiments, the subject in the present application is a human or a non-human primate.

The PI4KIIIα/FAM126/TTC7 complex inhibitor disclosed in the present application may be administered by routes of administration known in the art, such as injection (including subcutaneous injection, intraperitoneal injection, intravenous injection (drip or infusion), intramuscular injection, or intradermal injection), or non-injection (including oral administration, nasal administration, sublingual administration, rectal administration, or topical administration). In some embodiments, the PI4KIIIα/FAM126/TTC7 complex inhibitor in the present application is administered orally, subcutaneously, intramuscularly, or intravenously. In some embodiments, the PI4KIIIα/FAM126/TTC7 complex inhibitor in the present application is administered orally.

In the present application, the term "therapeutically effective amount" refers to an amount of a drug that can alleviate or eliminate a disease or symptom in a subject, or preventatively inhibit or prevent the occurrence of a disease or symptom. The therapeutically effective amount may be an amount of a drug that alleviates one or more diseases or symptoms of a subject to a certain extent; may be an amount of a drug that partially or fully restores one or more physiological or biochemical parameters associated with the cause of a disease or symptom to normal; and/or may be an amount of a drug that reduces the likelihood of a disease or symptom. In some embodiments, the term "therapeutically effective amount" refers to an amount of a drug that can alleviate or eliminate a mood disorder (such as anxiety or depression) in a subject.

The therapeutically effective amount of the PI4KIIIα/FAM126/TTC7 complex inhibitor provided by the present application depends on various factors known in the art, such as weight, age, past medical history, current received treatment, strength, allergies, hypersensitivity, and side effects of interaction between health status and drugs, route of administration, and extent of disease progression. A person skilled in the art (such as a physician or veterinarian) may correspondingly reduce or increase the dose according to these or other conditions or requirements.

In some embodiments, the method further comprising diagnosing the subject as having the mood disorder before administering an effective amount of PI4KIIIα/FAM126/TTC7 complex inhibitor to the subject. A subject may be diagnosed with a mood disorder by conventional diagnostic methods and criteria used by a psychiatric clinician.

In some embodiments, the treatment further includes administering a second reagent to the subject in need thereof.

In some embodiments, the second reagent is used for treating the mood disorder, including but not limited to, a tricyclic antidepressant, a selective serotonin reuptake inhibitor (SSRI), or a serotonin-norepinephrine reuptake inhibitor (SNRI).

In some embodiments, the second reagent is used for treating a neurodegenerative disease, stroke, or malignant tumor. In some embodiments, the reagent used for treating a neurodegenerative disease includes, but is not limited to, levodopa, carbidopa, selegiline, rasagiline, entacapone, tolcapone, amantadine, memantine, riluzole, bromocriptine, pergolide, apomorphine, ropinirole, pramipexole, atropine, scopolamine, trihexyphenidyl, benzatropine, procyclidine, tacrine, donepezil hydrochloride, galanthamine, huperzine A, and rivastigmine. In some embodiments, the reagent used for treating a stroke includes, but is not limited to, dipyridamole, low-molecular weight dextran, urokinase, heparin, and aspirin. In some embodiments, the reagent used for treating a malignant tumor includes, but is not limited to, cyclophosphamide, thiotepa, carmustine, cisplatin, carboplatin, methotrexate, pemetrexed, capecitabine, cytarabine, gemcitabine, paclitaxel, docetaxel, and vincristine.

In some embodiments, the PI4KIIIα/FAM126/TTC7 complex inhibitor is administered before, after, or simultaneously with the second reagent.

The present application also involves use of the PI4KIIIα/FAM126/TTC7 complex inhibitor in manufacture of a medicament for preventing or treating a mood disorder and use of the PI4KIIIα/FAM126/TTC7 complex inhibitor in preventing or treating a mood disorder.

### Drug screening

The present application further provides a method of screening a PI4KIIIα/FAM126/TTC7 complex inhibitor, including allowing a drug candidate to be in contact with PI4KIIIα, FAM126, TTC7, or EFR3a protein or nucleic acid, or PI4KIIIα/FAM126/TTC7 complex, and detecting whether the drug candidate can inhibit the formation or activity of the PI4KIIIα/FAM126/TTC7 complex.

The present application further provides a method of screening a drug for preventing or treating a mood disorder, including allowing a drug candidate to be in contact with PI4KIIIα, FAM126, TTC7, or EFR3a protein or nucleic acid, or PI4KIIIα/FAM126/TTC7 complex, and detecting whether the drug candidate can inhibit the formation or activity of the PI4KIIIα/FAM126/TTC7 complex.

### Examples

### Example 1: Antidepressant effect of orally administered PAO on chronic unpredictable moderate stress model in rats

### 1.1 Experimental principle

The chronic unpredictable moderate stress (CUMS) model is a model of depression in rats proposed by Willner et al. in 1987, inspired by the chronic unpredictable stress described by Katz, and has been appropriately modified. It is widely used in the current literature. This model more realistically simulates the "difficulties" people may encounter in their daily lives. In this model, the variability and unpredictability of stressors is the key to model making. More than 10 stressors are randomly selected during the whole experiment, so that animals cannot expect the occurrence of stimulation. This model is highly effective, basically meets the requirements of the depression model, and can be used to handle problems that cannot be resolved by other models. After long-term stimulation of mild stress, the reduction in consumption of sucrose water reflects the central symptom of endogenous depression, that is, anhedonia, and symptoms of other major depressive disorders are also simulated, such as the decline of athletic ability and social interaction ability, the decline of exploration ability, the defect of aggressive attack ability, and the decline of sexuality. Chronic stress-mediated behavioral abnormalities may persist for months, and long-term use of antidepressants can correct these abnormalities, while other types of psychotropic drugs are ineffective.

### 1.2 Experimental purpose

The purpose of this experiment is to observe the effect of oral administration of PAO on the preference for sugar water in the chronic unpredictable moderate stress rat model, to determine whether PAO has a good antidepressant effect.

### 1.3. Experimental materials

### 1.3.1 Experimental sample

Name: PAO
Preparation method: A required amount of PAO was weighed to prepare a stock solution of a concentration of 30 mg/mL with dimethyl sulfoxide (DMSO), and then the stock solution was diluted with water.
Oral administration by gavage: with a dose in a range of 0.1-3.0 mg/kg and a volume of 0.5 mL per 100 g of weight.

### 1.3.2 Positive control

Name: Venlafaxine Hydrochloride Capsules (Effexor XR)
Specification: 150 mg/capsule
Preparation method: Two venlafaxine hydrochloride capsules (150 mg/capsule) were crushed and then suspended in 0.5% CMC-Na under ultrasonication to be precisely adjusted in volume with 0.5% CMC-Na to a final volume of 100 mL.

### 1.3.3 Experimental animal

Species: Wistar rat
Number and sex: 60, male
Weight: 200-220 g (before grouping)
Source: Shanghai Silaike Experiment Animal Co., Ltd.

### 1.4 Experimental method

### 1.4.1 Animal grouping

After acclimated to the environment for five days, healthy SD rats with a weight of 220-250 g were selected for experiment and grouped according to the following Table 1.

**Table 1. Animal grouping**

| Group | Dose (mg/kg) | Number of animals |
|---|---|---|
| Blank (drinking water) | 0 | 10 |
| CUMS model (drinking water) | 0 | 10 |
| Positive drug venlafaxine | 15 | 10 |
| High dose of PAO by gavage | 3.0 | 10 |
| Medium dose of PAO by gavage | 1.5 | 10 |
| Low dose of PAO by gavage | 0.75 | 10 |

### 1.4.2 Animal modeling and administration method

### 1.4.2.1 Basic detection of sugar water preference

The rats were reared adaptively for one week and then subjected to the basic experiment of sugar water preference. For the first 24 h, all rats were given 1% sucrose water in two bottles placed in the left and right corners of a cage. For the next 24 h, all rats were given 1% sucrose water and normal drinking water respectively in two bottles which were still placed in the left and right corners of the cage. After that, all rats fasted for solids and liquids for 23 h and then subjected to the experiment of sugar water preference: all rats were given 1% sucrose water and normal drinking water, the amounts of the sucrose water and the normal drinking water consumed by the rats within 1 h were respectively measured by weighing the bottles. The rats were evenly grouped as shown in the Table 1 according to the weight, liquid consumption, and sugar water consumption, and then subjected to chronic unpredictable moderate stress ("CUMS").

### 1.4.2.2 CUMS depression modeling

CUMS depression modeling was carried out on all the groups except the blank group. More than 10 stressors were alternately given to the rats every week, so that the rats cannot predict the duration of each stimulation by a stressor and the stimulation to be given next. The stressors for the experiment were as follows: two times of flash for 7 h; one time of unclean litter environment (pour 150 mL of water on the litter) for 17 h; one time of intermittent noise for 3 h and one time of intermittent noise for 5 h; one time of night light (lasting for 36 h); one time of cage tilting for 7 h and one time of cage tilting for 22 h; one time of rearing in groups (five rats in one cage) for 17 h; one time of rearing in an environment with unfamiliar odor for 17 h; one time of rearing in an environment with foreign matter for 7 h; three times of fasting for solids for 19 h, 22 h, and 24 h respectively, with limited feed supply for 2 h after each fast; and four times of fasting for liquids for 17 h, 19 h, 20 h, and 21 h respectively, with an empty bottle placed for 1 h after each fast (table 2).

**Table 2. Schedule for CUMS stimulation**

| | Time | Schedule for stimulation (the type and duration of the stimulation are unchanged, and the schedule may be adjusted as the experiment progresses) |
|---|---|---|
| **Monday** | 9:00 | Rear in a single cage, administer drugs |
| | 11:00 | Supply food, clip tails |
| | 12:00 | Stop tail clipping, put a camphorwood block in, tilt the cage |
| **Tuesday** | 10:00 | Remove the camphorwood block, place foreign matter (a glass or plastic cup) in, administer drugs |
| | 16:00 | Remove water, food, and foreign matter, keep light on (switch on) |
| **Wednesday** | 9:00 | Provide white noise for 3 h, administer drugs |
| | 12:00 | Provide food and water, provide ice pellets (200 mL)/cage |
| **Thursday** | 9:00 | Change cages, provide white noise for about 7 h, weigh after administration, fast for solids and liquids |
| | | Prepare sugar water, provide a bottle of water and a bottle of sugar water in each cage, weigh the bottles |
| | 16:00 | Tilt the cage, turn on the flash |
| **Friday** | 9:00 | Turn off the flash, supply water and sugar water (to rats 1-5) for 1 h, weigh the bottles |
| | 10:30 | Supply water and sugar water (to rats 6-10) for 1 h, weigh the bottles |
| | 11:30 | Supply food, provide white noise for 6 h Administer drugs, wash and disinfect the bottles in the animal laboratory |
| | 16:00 | Stop the white noise, keep night light on (switch on) |
| **Saturday** | 10:00 | Supply water, administer drugs, tilt the cage |
| | 16:00 | Remove water, stop tilting the cage, turn on the flash |
| **Sunday** | 9:00 | Turn off the flash, supply water, administer drugs, remove food, provide hot water at 45-50°C (150 mL/cage) |
| | 16:00 | Rear five rats in one cage |

### 1.4.2.3 Administration method

During the first two weeks of CUMS modeling, PAO was not administered to the rats. At the first week after two weeks of CUMS modeling, PAO or venlafaxine was administered to the rats orally. At the same time, drinking water was given to the rats in the blank group and the model group. The administration was carried out once a day at a dose shown in the Table 1 until the end of CUMS modeling. CUMS stimulation was continuously carried out during the administration.

### 1.4.3 Depressive behavior indicator-detection of preference for sugar water

For every week, after fasting for solids and liquids, the liquid consumption was measured (on Friday), and for each rat, the weight (recorded on Thursday), total fluid intake, water intake, and sugar water intake were recorded. For each rat, the percentage of preference for sugar water (calculated as follows: [preference = (sugar water intake/total fluid intake) × 100%]) and the sugar water consumption per gram of weight were calculated. This experiment was carried out in a period of nine weeks.

### 1.4.4 Statistical method

Statistical analysis was carried out by using the *SPSS* software with data expressed as mean ± standard error (x̅ ± s.e.m.). The weight difference between the rats in the blank group and the rats in other groups was statistically analyzed by Two way ANOVA. After one week and two weeks of administration, the difference in the preference for sugar water between the model group and other groups and the difference in the preference for sugar water between the blank group and other groups were tested for significance by Unpaired t test, with ^{∗} indicating P < 0.05 and ^{∗∗} indicating P < 0.01.

### 1.5 Experimental result

### 1.5.1 Change in weight after CUMS modeling and administration

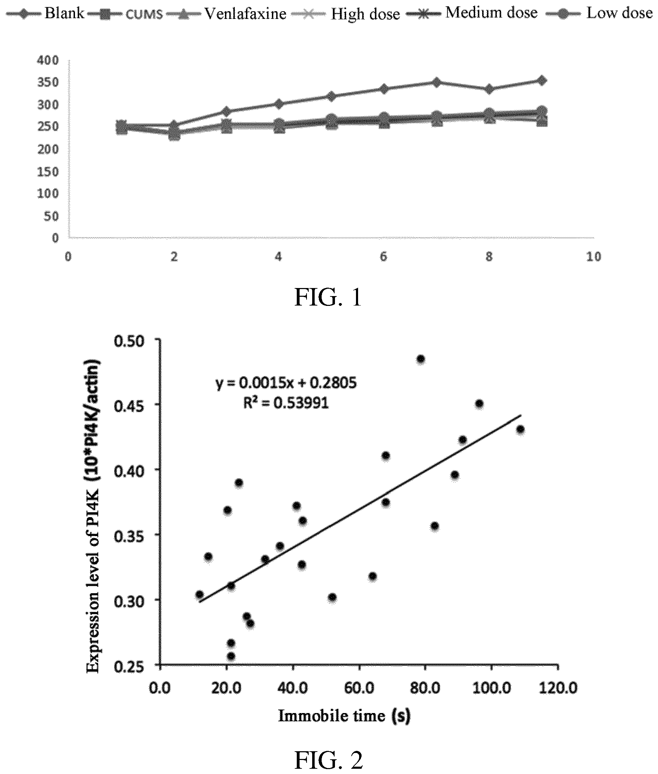

At the initial grouping, weights of rats in all groups are similar with no significant difference. With the progress of CUMS, except for the untreated blank group, the weight gain of rats in other groups is significantly slowed down due to CUMS modeling (Table 3, FIG. 1). Since the first week of stimulation, the weight of rats in other groups stimulated by CUMS is lower than that of rats in the blank group in weekly measurements (Table 3, FIG. 1), indicating that the use of CUMS for depression modeling in this experiment is very effective, greatly simulating the weight loss during the onset of depression.

**Table 3. Weight of rats in each group during CUMS modeling and administration**

| Time | Blank control | Model control | Venlafaxine | High dose of PI | Medium dose of PI | Low dose of PI |
|---|---|---|---|---|---|---|
| Initial grouping | 251.8 | 245.2 | 251.1 | 253 | 250.9 | 251.4 |
| CUMS for 1 week | 251.7 | 232.2 | 234 | 233 | 234.9 | 235.2 |
| CUMS for 2 weeks | 282.6 | 247.1 | 252.3 | 247.6 | 254.4 | 254.4 |
| CUMS for 3 weeks | 299.7 | 245.8 | 251.5 | 248.1 | 252.3 | 255.5 |
| CUMS for 4 weeks | 316.9 | 256.5 | 264.3 | 261.3 | 260 | 266 |
| CUMS for 5 weeks | 333.7 | 258 | 268.1 | 263.3 | 262.3 | 269.6 |
| CUMS for 6 weeks | 348.4 | 262.7 | 269.3 | 264.2 | 269.1 | 272.5 |
| CUMS for 7 weeks | 333.2 | 268.4 | 273.3 | 266.8 | 273.3 | 279 |
| CUMS for 8 weeks | 352.8 | 262.7 | 276 | 272.9 | 278.5 | 283.9 |

### 1.5.2 Antidepressant effect of multiple administration of PAO

At the initial grouping, rats in all groups have similar preference for sugar water. After six weeks of administration, both PAO and venlafaxine at doses of 3 mg/kg and 0.75 mg/kg can increase the preference for sugar water in rats, have significant difference (P < 0.05 or P < 0.01) from the model group, and have the preference for sugar water similar to that of the blank group (P > 0.05) (table 4). The results of this experiment show that, in the CUMS depression model of rats, oral administration of PAO has a significant antidepressant effect, and has an onset time slightly later than oral administration by gavage of venlafaxine.

**Table 4. Effect of PAO on preference for sugar water in CUMS rats (for each group, n=10, x̅ ± s.e.m.)**

| Time | Blank control | Model control | Venlafaxine | High dose of PAO | Medium dose of PAO | Low dose of PAO |
|---|---|---|---|---|---|---|
| CUMS for 5 weeks | 0.90±0.10 | 0.85±0.22 | 0.90±0.08 | 0.81±0.21 | 0.72±0.30 | 0.87±0.15 |
| CUMS for 6 weeks | 0.87±0.25 | 0.83±0.22 | 0.94±0.04^{∗} | 0.84±0.17 | 0.78±0.20 | 0.88±0.14 |
| CUMS for 7 weeks | 0.89±0.05^{∗} | 0.79±0.1 | 0.92±0.07^{∗∗} | 0.90±0.06^{∗} | 0.86±0.08 | 0.91±0.11^{∗} |
| CUMS for 8 weeks | 0.90±0.07^{∗} | 0.72±0.23 | 0.90±0.07^{∗} | 0.86±0.10 | 0.81±0.14 | 0.92±0.06^{∗} |
| Compared with the model group, ^{∗∗} P < 0.01, and ^{∗} P < 0.05. | | | | | | |

### 1.6 Conclusion

Oral administration of PAO or positive drug venlafaxine at doses of 3 mg/kg or 0.75 mg/kg once a day for 6 weeks can increase the preference for sugar water in the CUMS depression rat model to a level similar to normal rats, reflecting the antidepressant effect. Oral administration of PAO at a dose of 1.5 mg/kg can also increase the preference for sugar water.

### Example 2: Further verification and mechanism study of the anti-anxiety and anti-depression effect of PAO

### 2.1 Strain, breeding, and reproduction of mice

The mouse strains used in the present invention include C57/6B and ICR wild-type mice, Efr3a^{-/+} mice (the second exon of Efr3a is unconditionally knocked out and then results in a subsequent genetic code shift), Pi4kaGt(RRO073)^{Byg/+} (Pi4k^{-/+} for short) mice, and APP/PS 1 mice.

The Pi4kaGt(RRO073)^{Byg/+} mice were obtained by backcrossing Pi4kaGt(RRO073)^{Byg/+}] mice (MMRRC, Cat. #016351-UCD) with C57/6B mice from the Mutant Mouse Resource and Research Center for more than five generations. In this kind of mice, a transposon was inserted into the PI4KA gene (encoding PI4KIIIα) and polyA was introduced, so that the transcription of PI4KA can be prematurely terminated. The APP/PS 1 mice are double-transgenic mice with the genotype of B6.Cg-Tg (APPswe, PSEN1dE9) 85Dbo/Mmjax (MMRRC ID 034832-JAX), which are mice in a model of Alzheimer's disease (AD).

In the present application, mice were reared in standard sized cages of 5 per cage, with occasional 4 or 6 per cage. Unless otherwise specified, mice were allowed to eat and drink freely.

### 2.2 Establishment of hormone-induced mouse model of anxiety and depression

ICR mice were administered with corticosterone (GC, also known as 11β,21-dihydroxyprogesterone) orally for one month to obtain a mouse depression model. An aqueous solution with a GC concentration of 35 µg/mL was prepared with 0.45% hydroxypropyl-β-cyclodextrin (β-CD) as a solubilizing agent.

60 male C57B/6 WT mice were divided into six groups, 10 in each group. When the mice were 3 months old, the drinking water of four groups of mice was replaced by the aqueous GC solution, and the other two groups continued to be given normal drinking water, and the weight changes and water intake of the mice were recorded weekly. After one month, two groups were randomly selected from the mice fed with the GC solution and water separately, and administered with PAO solution by gavage. PAO was administered at regular times daily by gavage at doses of 0.2 mg/kg and 0.4 mg/kg in a total volume of 300 µL. After one month of the administration, that is, when the mice were 5 months old, the behavioral experimental analysis of the mice was carried out.

### 2.3 Preparation of PAO or its derivatives

Preparation of PAO stock solution: ≤ 30 mg of PAO was added into 1.0 mL of pure DMSO solvent, and then sonicated or vibrated until PAO was completely dissolved. Then, the mixture was diluted with water to a required concentration and a final DMSO concentration of 0.1%.

In some examples, PAO or its derivatives are dissolved in medium-chain triglycerides to obtain a stock solution with a concentration of 1.0 mg/g, and the stock solution is diluted with medium-chain triglycerides to a specific concentration for gavage.

### 2.4 Mouse behavioral experiment method

### 2.4.1 Forced swimming test (FST)

In the Forced swimming test, experimental animals are placed in a confined environment (such as water) in which they struggle desperately to escape but cannot escape, thus providing an unavoidable oppressive environment. After a period of time, the animals exhibit a typical "immobile state". This is a model of depression known as a "behavioral despair state". The experiment is carried out in a transparent plastic cylinder with an inner diameter of 15 cm and a height of 30 cm. The height of water injection is 15 cm, which can be adjusted properly to the height where the tail of a mouse cannot touch the bottom and the mouse cannot jump out of the cylinder. During the experiment, the water temperature should be kept at 22-25°C. A forced swimming image acquisition system should be placed in front of the FST device at a certain distance from the cylinder where the movement of the limbs of the mouse in the water can be clearly recorded. Each FST needs to carry out for 6 min. The immobile time of the mouse from the second minute to the sixth minute is analyzed. After the experiment, the mouse is taken out in time and dried. If the mouse is drowning during the experiment, it should be taken out in time and the FST experiment should be stopped. During the experiment, an absolutely silent environment should be created and maintained. The longer the mouse remains immobile for a certain period of time, the more depressed the mouse is.

### 2.4.2 Light/dark box (LDB) test

The light/dark box test is a test of anxiety behavior assessment in animals based on the dark addiction (dark box) and the exploratory trait (light box) of the animals. The LDB is carried out by using a whole rectangular box (45×27×27 cm) without a cover consisting of a light box (27×27 cm) with nine squares in the same size and a dark box (18×27 cm) with six squares in the same size. The light box and the dark box are separated by a panel with a small window (7.5×7.5 cm). An image acquisition system is placed directly above the box to record, within a set time, the distance and trajectory of a mouse between the light box and the dark box, the number of times of entering and leaving the light box and the dark box, and the time spent in the light box or the dark box. The more frequently the mouse enters and leaves the light box and the dark box, the longer the distance between the boxes is, or the longer the time spent in the dark box is, the more anxious the mouse is. Before the experiment, the cage should be wrapped with tin foil but with an opening for air exchange. The experiment should be started after shading for half an hour. The purpose is to allow the mouse to adapt to the dark environment in advance and eliminate the experimental panic and anxiety state of the mouse. A mouse is placed in the center of the light box or dark box, facing the small window. The number of times of entering and leaving the light box and the dark box, the distance between the light box and the dark box, and the time spent in each box, within 5 min 30 s, are recorded. Behavioral analysis is carried out after image acquisition. After each experiment, areas that the mouse touched in the light and dark box are thoroughly wiped with 70% ethanol, and the experiment of a next mouse is started after the odor of ethanol is completely dissipated, so as to avoid unnecessary misleading and interference in the behavior of the latter mouse by the scent of the former mouse. During the experiment, an absolutely silent environment should be created and maintained.

### 2.4.3 Elevated plus maze (EPM) test

The Elevated plus maze is one of the experimental methods for evaluating anxiety responses in rodents. Compared with the detection of anxiety behavior in mice caused by noxious stimuli (such as electrical stimulation, noise stimulation, dietary deprivation, and exposure to predator odors, etc), this experiment has the advantages of simple operation and less noxiousness, and can intuitively reflect the conditioned response of mice. The EPM consists of two open arms and two closed arms that cross in a cruciform shape, and the intersecting part is the central area about 75 cm above the ground. The principle is that in the face of new things (open arms) mice will develop curiosity to explore, but they also have a dark-addictive nature, and the conflict behavior between exploration and avoidance results in anxiety. During the experiment, a single mouse is placed in the central area facing the closed arm, and the number of times entering and leaving the open arm and closed arm, and the time spent in each arm, within 5 min 30 s, are recorded. The more frequently the mouse enters the open arm and closed arm, or the longer the time spent in the closed arm is, the more anxious the mouse is. After each experiment, areas that the mouse touched in the EPM are thoroughly wiped with 70% ethanol, and the experiment of a next mouse is started after the odor of ethanol is completely dissipated, so as to avoid unnecessary misleading and interference in the behavior of the latter mouse by the scent of the former mouse. During the experiment, an absolutely silent environment should be created and maintained.

### 2.4.4 Novelty-suppressed feeding (NSF) test

Novelty-suppressed feeding test is to detect the conflict between feeding in a novel environment and fear of the novel environment in rodents after fasting and starvation. The experimental site is a white opaque plexiglass box of 30×30 square, 20 cm high, with a piece of normal food for mice placed in the center of the bottom of the box. After fasting for 24 h, a single mouse is randomly placed in a corner of the box with the head toward the corner of the box. The activity of the mouse in the box is observed until the mouse starts to eat the food. Then, the mouse is taken out, and the time from being placed in the box to starting to eat the food (feeding latency) is recorded. Unless otherwise designed or specified, if the mouse does not eat the food in 5 min, the mouse is also taken out, and the feeding latency of the mouse is recorded as 5 min. The longer the feeding latency is, the more anxious the mouse is.

### 2.4.5 Chronic unpredictable moderate stress (CUMS) model

The experiment lasts for 8-10 weeks. Two of various stressors are randomly selected and given to mice every day. The stressors include: tail clipping for 15 min, strong light stimulation for 4 h, noise for 10 min, cage tilting at 45° for 24 h, hanging upside down for 10 min, restriction on activity space for 4 h, unclean litter environment (pour 150 mL of water on the litter) for 24 h, night light (for 36 h), environment with unfamiliar foreign matter for 7 h, fasting for solids for 24 h (remove litter during fasting), and fasting for liquids for 24 h (remove litter during fasting). However, the fast for liquids is carried out only once a week, and does not intersect with the unclean litter environment. After the fast for liquids, each mouse is weighed, and the preference for sugar water is measured. For the measurement of the preference for sugar water, a single mouse is placed in a new cage in which two bottles weighed in advance are placed, one containing pure water and the other containing 1% sugar water. After the mouse drinks for 15 min, the two bottles are exchanged, and then the mouse drinks for 45 min. The two bottles are taken out and weighed separately. Immediately after the experiment, the mouse is provided with sufficient water and food.

### 2.4.6 Morris water maze (MWM) test

The Morris water maze test device is composed of three parts: water maze, water maze image acquisition system, and software analysis system. The water maze is mainly composed of a water pool (diameter = 1.2 m) and a circular transparent platform (diameter = 5 cm) that is adjustable in height and removable. The water pool contains titanium dioxide, which should be stirred and mixed uniformly before the start of each experiment so that the mouse cannot intuitively find the platform in the water pool. The water temperature is kept at about 20°C during the experiment. The behavior of mouse in the water pool is acquired by a camera placed above the water maze, and transmitted to a computer connected to the camera, for analysis and storage on-line or off-line.

The Morris water maze test mainly consists of three phases: adaptive training, acquisition training, exploration training, and sometimes reverse acquisition training. The brief steps are as follows:

Adaptive training: The day before the start of acquisition learning, the water is adjusted to a proper temperature and mixed uniformly, and a platform 0.5 cm above the water surface is placed in the third quadrant of the water pool. A mouse, facing the pool wall, is placed in the water pool to allow it swim freely for 1 min, and then the mouse is taken out and wiped dry. If the mouse can climb on the platform within 1 min, the mouse is allowed to stay on the platform for 0.5 min.

Acquisition training: usually lasts for 7 days, but may be longer or shorter. The water pool is divided into four quadrants, and a platform is placed in the middle of the first quadrant. The mouse facing the pool wall is randomly placed into water from each of the four quadrants. Four different markers of triangle, square, four-pointed star, and circle are respectively pasted on the walls of four quadrants of the water pool to facilitate the memory and learning of the mouse. The time it takes for the mouse to find the platform underwater is recorded. If the time exceeds 60 s, the mouse is guided to the platform and allowed to stay on the platform for 30 s, and then taken out and wiped dry. If the mouse finds the platform before 60 s, the time is recorded, and the mouse is allowed to stay on the platform for 30 s, and then taken out and wiped dry. If the mouse has the ability to learn and remember, the time it takes to find the platform will be shorter and shorter every day.

Exploration training: The day after the last acquisition training, the platform is removed from the first quadrant to start 60 seconds of exploration training. The mouse is put into water from the third quadrant or the fourth quadrant (farthest from the first quadrant), and the percentage of time spent by the mouse in the first quadrant (the quadrant where the platform was placed before) is recorded as a detection indicator of spatial memory. After the experiment is terminated, the mouse should be taken out in time and wiped dry. Generally, the mouse that can spend the least time in each quadrant to find the platform in the acquisition training or that has a high percentage of time spent in the first quadrant in the exploration training has a strong learning and memory ability. During the experiment, an absolutely silent environment should be created and maintained.

Reverse acquisition training: After the exploration phase, the platform is placed in the quadrant contralateral to the original position, and the training is carried out on each mouse in four quadrants per day for a total of four times, each time for 60 s. After a mouse finds the hidden platform, it is allowed to stay on the platform for 30 s. If the mouse does not find the platform, it is guided to the platform and allowed to stay on the platform for 30 s. A training interval between mice is 15-20 min.

### 2.5 Quantitative PCR analysis of mRNA level of PIK4IIIα in mouse brain

### 2.5.1 Main equipment and reagents

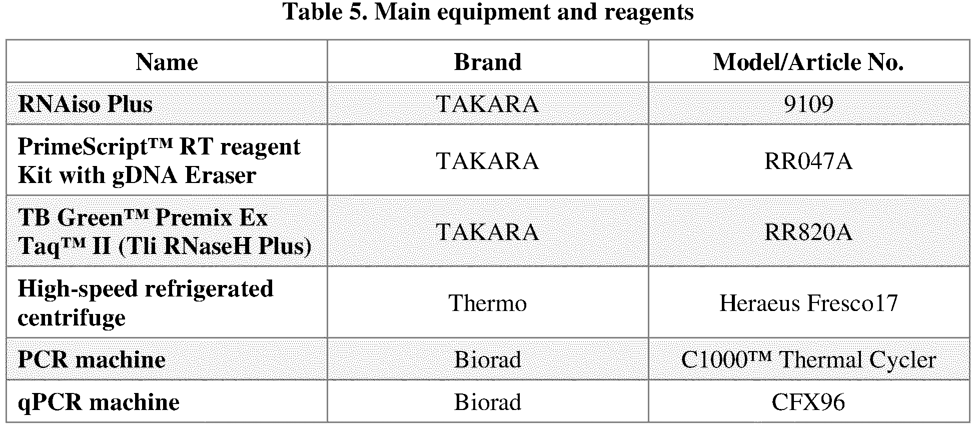

### 2.5.2 Primer information

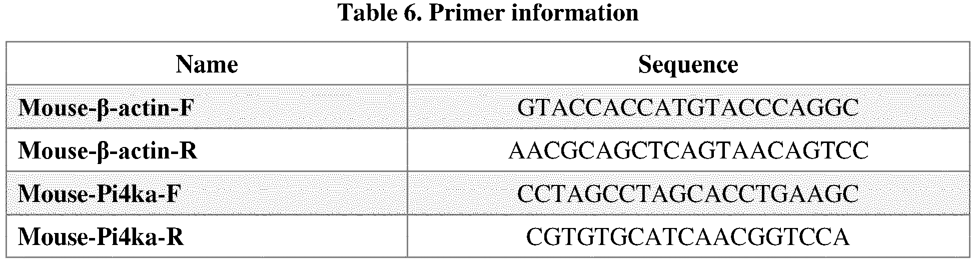

### 2.5.3 Experimental steps

RNA extraction: 1000 µL of RNAiso Plus was added into each brain tissue sample (half brain), then ground with a tissue lyser (10 r/s × 10 s/cycle × 10 cycles), allowed to stand at room temperature for 5 min, and centrifuged at 12000 g at 4°C for 10 min. 900 µL of supernatant was taken out and added into a new 1.5 mL EP tube, then 200 µL of chloroform was added and mixed uniformly, allowed to stand at room temperature for 5 min, and centrifuged at 12000 g at 4°C for 15 min. 400 µL of supernatant was transferred into a new 1.5 mL EP tube, then 800 µL of isopropanol was added and allowed to stand at room temperature for 10 min, and centrifuged at 12000 g at 4°C for 10 min, and then the supernatant was removed. The precipitate was added into a tube containing 1 mL of 75% ethanol and mixed uniformly, then centrifuged at 7500 g 4°C for 5 min, and then the supernatant was removed. The precipitate was dried at room temperature, and then dissolved with 50 µL of H₂O.

Reverse transcription: A DNA-free reverse transcription kit with a 20 µL system was used. 1 µg of RNA, 2 µL of 5×gDNA eraser buffer, and 1 µL of gDNA eraser were added into a 200 µL PCR tube, and H₂O was then added to make up to 10 µL. The digestion of DNA was carried out at 42°C for 2 min. 1 µL of PrimeScript RT Enzyme MIX I, 1 µL of RT primer mix, 4 µL of 5×primeScript buffer2 (for real time), and 4 µL of RNase free H₂O were added into the reaction solution. The reverse transcription was carried out according to the procedure of 37°C for 15 min, 85°C for 5 s, 4°C. The obtained cDNA was used immediately or stored at -20°C for use.

Real-time quantitative PCR: The cDNA was diluted 10-fold with RNase free H₂O. The reaction system was 12.5 µL of TB Green Premix Ex Taq II, 1 µL of forward and reverse primers (10 µM) each, 2 µL of cDNA template, and 8.5 µL of RNase free H₂O, a total of 25 µL. The reaction process includes two steps: initial denaturation at 95°C for 30 s; PCR reaction at 95°C for 5 s and at 60°C for 30 s, a total of 40 cycles. Finally, the procedure of plotting a melting curve was run. Each sample underwent three technical replicates.

**2.5.4 Data analysis:** The relative quantification was carried out for target genes by ΔCT method.

### 2.6 Data analysis and statistics

Comparative analysis of data is carried out by using the SPSS software. The data statistics in the article is presented in the form of mean ± standard error. The criterion for data to have significant difference is p < 0.05.

### 2.7 Experimental results

### 2.7.1 The mRNA level of PIK4IIIα in mouse brain is positively correlated with the degree of depression

35 male ICR mice aged 2 months were subjected to the first forced swimming test to experience unavoidable oppression. On the seventh day afterward, the mice were subjected to the second forced swimming test, and the time each mouse was in the "immobile state" during the last 4 min of the total 6 min of the second forced swimming test was recorded. Then, the brain of each mouse was quickly removed, and the total RNA in the brain tissue was extracted and reverse transcribed to obtain cDNA. For each mouse, the quantitative PCR was carried out on cDNA of PI4KIIIα and internal reference beta-actin, and the amount of RNA of PI4KIIIα in the brain tissue relative to the internal reference RNA was calculated and analyzed by the ΔCT method. After excluding the data of the time for the "immobile state" less than 10 s or greater than 160 s, a scatter plot was made with the remaining time for the "immobile state" as the X-axis value and the relative amount of RNA of PI4KIIIα as the Y-axis value (FIG. 2). The results show that the mRNA level of PIK4IIIα in mouse brain is linearly positively correlated with the degree of depression. This experimental conclusion indicates that PI4KIIIα plays a role in the regulation or mediation of depression severity.

### 2.7.2 The PI4KIIIα inhibitor alleviates anxiety and depressive behaviors in APP/PS1 mice

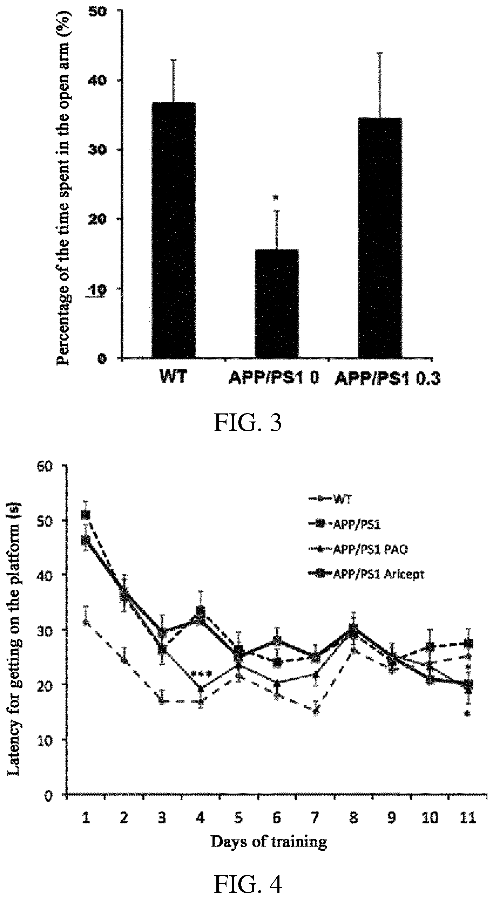

There were two groups of mice: nine male APP/PS 1 mice aged 6 months and nine male wild-type littermates. The two groups of mice were administered with a PAO solution (at a dose of 0.3 mg/kg weight) by gavage once a day from Monday to Friday, and skipped on Saturday and Sunday. Another nine male APP/PS 1 mice of the same age were administered with a solution without PAO by gavage at the same time. Two months later, the three groups of mice underwent eight days of MWM training followed by a day of memory test for other experimental purposes. When the mice were 10 months old, each mouse was transferred into an opaque cover-free PMMA box with a length, width, and height of 15 cm, with a modified household electric mosquito swatter placed at the bottom of the box. The mouse was given a one-second electric shock when it was in the center of the swatter. Then, the mouse was placed back to a home cage. After 7 days, the mice were subjected to the EPM test, the time spent in the open arm of each mouse was recorded, and the percentage of the time spent in the open arm in the total time spent in the EPM was calculated. As shown in FIG. 3, the percentage of the time spent in the open arm of the APP/PS 1 mice without PAO treatment is significantly less than that of the wild-type mice, and the percentage of the time spent in the open arm of the APP/PS 1 mice with PAO treatment is not significantly different from that of the wild-type mice. As shown in FIG. 3, PAO can prevent or alleviate anxiety symptoms in APP/PS 1 mice.

To explore whether there is a necessary interdependence between the therapeutic effect of drugs on anxiety behavior in APP/PS 1 mice and the therapeutic effect of drugs on learning and memory impairment in APP/PS 1 mice, the therapeutic effect of PAO and Aricept (donepezil hydrochloride) on the anxiety and the learning and memory impairment in APP/PS 1 mice was tested. 32 male APP/PS 1 littermate mice aged 4 months, the offspring of APP/PS 1 mated with C57B/6, were randomly divided into two groups, one group of mice (referred to as APP/PS 1 PAO mice) were administered with PAO (at a dose of 0.1 mg/kg weight) by gavage once a day from Monday to Saturday, and the other group of mice (referred to as APP/PS 1 Aricept mice) were administered with Aricept (at a dose of 1.0 mg/kg weight) by gavage once a day from Monday to Saturday. In addition, 16 WT mice and 20 APP/PS 1 mice that were male littermate aged 4 months were administered with 0.1% aqueous DMSO solution by gavage. After five weeks, the MWM test was carried out. As shown in FIG. 4, the APP/PS 1 mice without treatment perform the worst in the water maze, and the WT mice perform the best. The mice with treatment by PAO perform significantly better than the APP/PS 1 mice without treatment in both the acquisition training and the reverse acquisition training, indicating that PAO significantly improves the learning and memory ability of APP/PS 1 mice. The mice with treatment by Aricept perform with no significant difference from the APP/PS 1 mice without treatment in the acquisition training, but perform as well as the mice with treatment by PAO in the reverse acquisition training, better than the mice without treatment, indicating that Aricept significantly improves the learning and memory ability of APP/PS 1 mice, but has a treatment effect not as good as PAO.

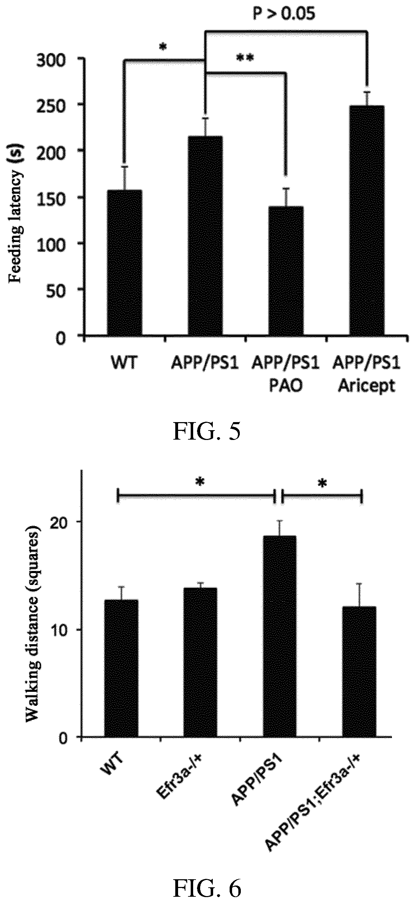

One week after the MWM test, the mice were subjected to the NSF test. As shown in FIG. 5, the APP/PS 1 mice without treatment have a feeding latency significantly longer than the WT mice, and the APP/PS 1 mice with treatment by PAO have a feeding latency not significantly different from the WT mice, indicating that PAO can significantly reduce the anxiety level of APP/PS 1 mice; however, the APP/PS 1 mice with treatment by Aricept have an anxiety level not significantly different from or even higher than the APP/PS 1 mice without treatment, indicating that Aricept can significantly improve the learning and memory ability of APP/PS 1 mice, but cannot reduce the anxiety level of APP/PS 1 mice. Therefore, the improvement of learning and memory is not necessarily related to the reduction of anxiety level.

### 2.7.3 Downregulation of PI4KIIIα in wild-type mice can prevent or alleviate depression and anxiety manifestations

15 2-month-old WT mice and 15 2-month-old Pi4k^{-/+} mice, the offspring of Pi4k^{-/+} mated with C57/6B, were transferred to the behavioral laboratory for 1 week of adaptation. The CUMS experiment was started from the second week. From the third week of CUMS, the WT mice had a weight gain stopped or even had a weight decrease, while the Pi4k^{-/+} mice had a continuous weight gain. From the sixth week of CUMS, the Pi4k^{-/+} mice are significantly different from the WT mice (table 7). For the preference of sugar water, before CUMS and during the first two weeks of CUMS, the two groups of mice had a significant preference for sugar water and had no significant difference from each other. From the third week of CUMS, the two groups of mice have the preference for sugar water decreased week by week, and the WT mice have the preference for sugar water decreased faster than the Pi4k^{-/+} mice; and at the seventh week of CUMS, the WT mice have the preference for sugar water significantly lower than the Pi4k^{-/+} mice (table 8). The experimental results show that downregulation of PI4KIIIα expression can prevent or alleviate depression symptoms.

On the day after the CUMS experiment, the mice were provided with sufficient water and food. On the second day, fasting for solids, water supply, and litter removal were started at 9:00 am. On the third day, the NSF test was carried out. The results show that the WT mice have a mean feeding latency significantly longer than the Pi4k^{-/+} mice (WT: 212±45 s, Pi4k^{-/+}: 154±27 s, T-Test, P<0.05). The experimental results show that downregulation of PI4KIIIα expression can prevent or alleviate anxiety symptoms.

**Table 7. Weight of WT and Pi4k^{-/+} mice during CUMS (n=15, mean ± s.e.m.)**

| Time | WT | Pi4K^{-/+} |
|---|---|---|
| Before CUMS | 24.5±1.2 | 24.3±1.6 |
| CUMS for 1 week | 25.1±1.1 | 24.8±1.4 |
| CUMS for 2 weeks | 25.2±1.5 | 25.2±1.7 |
| CUMS for 3 weeks | 25.4±1.7 | 25.8±1.5 |
| CUMS for 4 weeks | 25.6±3.0 | 26.3±1.3 |
| CUMS for 5 weeks | 25.2±2.2 | 26.8±1.6 |
| CUMS for 6 weeks | 25.0±1.7 | 27.2±2.1 ^{∗} |
| CUMS for 7 weeks | 24.4±2.2 | 27.5±1.6^{∗} |
| CUMS for 8 weeks | 24.1±1.3 | 27.5±1.5^{∗} |
| CUMS for 9 weeks | 23.5±2.0 | 27.7±1.0^{∗∗} |

| | | |
|---|---|---|
| "^{∗}" indicates that there is a significant difference from the corresponding WT mice, p<0.05 | | |

**Table 8. Effects of Pi4k deletion on preference for sugar water in CUMS mice (n=15, mean ± s.e.m.)**

| Time | WT | Pi4K^{-/+} |
|---|---|---|
| Before CUMS | 0.84±0.15 | 0.85±0.2 |
| CUMS for 1 week | 0.83±0.15 | 0.84±0.21 |
| CUMS for 2 weeks | 0.81±0.12 | 0.84±0.17 |
| CUMS for 3 weeks | 0.78±0.15 | 0.83±0.16 |
| CUMS for 4 weeks | 0.75±0.1 | 0.84±0.11 |
| CUMS for 5 weeks | 0.72±0.15 | 0.82±0.17 |
| CUMS for 6 weeks | 0.71±0.15 | 0.80±0.15 |
| CUMS for 7 weeks | 0.64±0.12 | 0.81±0.13^{∗} |
| CUMS for 8 weeks | 0.60±0.11 | 0.80±0.14^{∗} |
| CUMS for 9 weeks | 0.53±0.1 | 0.77±0.10^{∗∗} |

| | | |
|---|---|---|
| "^{∗}" and "^{∗∗}" indicate that there is a significant difference from the corresponding WT mice, p<0.05, p<0.01 | | |

### 2.7.4 Downregulation of PI4KIIIα can prevent or alleviate depression and anxiety manifestations in mice of neurodegenerative disease model

To test whether the downregulation of PI4KIIIα expression can prevent or alleviate other diseases, especially anxiety and depression associated with nervous system diseases, 15 "APP/PS1" mice aged 4 months and 15 "APP/PS1; Pi4k^{-/+}" mice aged 4 months, the offspring of Pi4k^{-/+} mated with APP/PS 1 with C57/6B gene background, were subjected to the CUMS experiment, and the preference for sugar water was measured for the two groups of mice weekly. As shown in Table 9, the APP/PS 1; Pi4k^{-/+} mice have a preference for sugar water decreased significantly slower than the APP/PS 1 mice, indicating that the downregulation of PI4KIIIα expression can prevent or alleviate the depression symptoms of APP/PS 1 mice.

**Table 9 Effects of downregulation of PI4KIIIα on preference for sugar water in APP/PS1 mice after CUMS (n=15, mean ± s.e.m.)**

| Time | APP/PS 1 | APP/PS 1; Pi4K^{-/+} |
|---|---|---|
| Before CUMS | 0.78±0.15 | 0.83±0.17 |
| CUMS for 1 week | 0.75±0.15 | 0.81±0.12 |
| CUMS for 2 weeks | 0.73±0.18 | 0.82±0.16 |
| CUMS for 3 weeks | 0.70±0.09 | 0.79±0.17 |
| CUMS for 4 weeks | 0.65±0.11 | 0.77±0.11 |
| CUMS for 5 weeks | 0.61±0.10 | 0.81±0.19^{∗} |
| CUMS for 6 weeks | 0.55±0.11 | 0.78±0.12^{∗} |
| CUMS for 7 weeks | 0.51±0.10 | 0.75±0.13^{∗∗} |

| | | |
|---|---|---|
| "^{∗}" and "^{∗∗}" indicate that there is a significant difference from the corresponding APP/PS1 mice, p<0.05, p<0.01 | | |

Similarly, on the day after the CUMS experiment, the mice were provided with sufficient water and food. On the second day, fasting for solids, water supply, and litter removal were started at 9:00 am. On the third day, the NSF test was carried out. The results show that the APP/PS1; Pi4K^{-/+} mice have a mean feeding latency significantly shorter than the APP/PS1 mice (APP/PS1: 242 s, Pi4k^{-/+}: 183 s, T-Test, P<0.05). The experimental results show that downregulation of PI4KIIIα expression can prevent or alleviate anxiety symptoms in APP/PS 1 mice.

### 2.7.5 Downregulation of Efr3a can prevent or alleviate depression and anxiety manifestations in mice of neurodegenerative disease model

There were 15 WT mice, 4 Efr3a^{-/+} mice, 14 APP/PS 1 mice, and 9 APP/PS 1; Efr3a^{-/+} mice, the offspring littermates of APP/PS 1 mated with Efr3a^{-/+}. When the mice were 8 months old, the LDB test was carried out. As shown in FIG. 6, the 8-month-old APP/PS 1 mice have a quantity of squares where the mice walk through in the light box (a walking distance) significantly increased compared with the WT mice, while the APP/PS 1; Efr3a^{-/+} mice of the same age have no significant difference from the WT mice, but have a walking distance significantly reduced compared with the APP/PS 1 mice. It indicates that the APP/PS 1 mice have an anxiety level significantly higher than the WT mice, and knocking out one copy of the Efr3a gene can significantly prevent or reduce the anxiety level of APP/PS 1 mice.

### Example 3: Inhibitory effect of PAO and its derivatives on anxiety and depression in mice

In this experiment, the inhibitory effect of the PI4KIIIa inhibitor PAO and its derivatives on anxiety and depression in wild-type mice with no apparent other disease symptoms or neurological damage was tested.

### 3.1 PAO derivatives inhibit depressive behavior in wild-type mice

The effects of PAO derivatives on ICR mice in the FST were also tested. For example, the PAO derivatives include PI04 formed by substituting -H at the para position of the benzene ring of PAO with -F, PI05 formed by substituting -H at the para position of the benzene ring of PAO with -CF₃, and PI06 formed by substituting -H at the para position of the benzene ring of PAO with -I. Each of these three PAO derivatives were first formulated with medium-chain triglyceride-MIGLYOL 812N (IOI Oleo GmbH) into a stock solution with a concentration of 1.0 mg/g. The mice were administered with the solution diluted to 0.033 mg/mL with the same medium-chain triglyceride in a three-step gradient by gavage every day at a dose of 0.3 mg/kg. A control group (veichle) was only administered with medium-chain triglyceride.

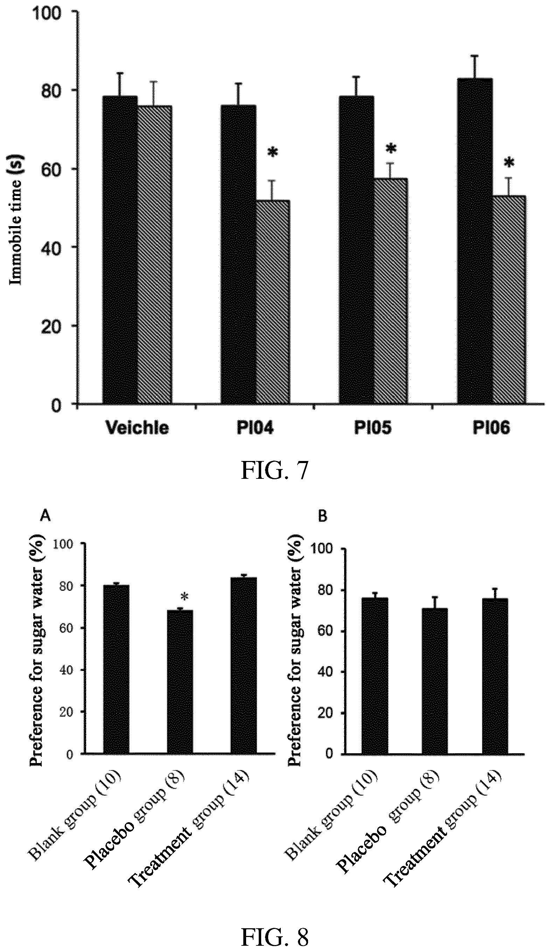

80 2-month-old ICR mice were randomly divided into four groups. After one week of adaptation in the forced swimming lab, the mice were subjected to the first 6-minute forced swimming, and the immobile time within the last four minutes was recorded. After excluding the data of the immobile time less than 10 s or greater than 160 s, there were 16 veichle (only administered with medium-chain triglyceride) mice, 14 PI04 mice, 17 PI05 mice, and 15 PI06 mice left. Then, each group of mice were administered by gavage at a dose of 0.3 mg/kg once a day for 6 days. On the seventh day, the mice were subjected to the second 6-minute forced swimming. Each mouse was administered in the same dose 60 minutes before the forced swimming. For each mouse, the immobile time within the last four minutes was recorded. As shown in FIG. 7, there is no significant difference in the immobile time between the first and second forced swimming experiments in the mice only administered with medium-chain triglyceride, and the mice in the PI04, PI05, and PI06 groups have the immobile time in the second forced swimming experiment significantly reduced compared with the first forced swimming experiment, indicating that the PAO derivatives PI04, PI05, and PI06 can alleviate the behavioral performance of anxiety and depression.

### 3.2 PAO prevents or alleviates anxiety and depression caused by neuronal damage

The small-molecule compound 1-methyl-4-phenyl-1,2,3,6-tetrahy-dropyridine (MPTP) is dehydrogenated by the monoamine oxidase B (MAO-B) to produce MPP⁺ ions. This ion inhibits mitochondrial complex I, leading to cytotoxicity and even death of the cell. The MPP⁺ ions are specifically taken up in a large amount by dopamine neurons in the substantia nigra. MPP⁺ blocks the complex I of the mitochondrial respiratory chain, leading to neurodegenerative changes in dopamine neurons.

50 male C57B6 mice aged 4 months were randomly divided into a blank group including 10 mice, a placebo (PD-ctrl) group including 20 mice with neuronal damage, and a PAO treatment (PD-PAO) group including 20 mice with neuronal damage. MPTP was first dissolved with DMSO and then diluted with saline. Probenecid was dissolved with 5% aqueous sodium bicarbonate solution. First, each mouse in the PD-ctrl group and the PD-PAO group was intraperitoneally injected with MPTP (supplier: CSN Pharm) at a dose of 25 mg/kg once a day for 5 days, with two days of drug withdrawal, and then injected for 5 days as in the first week. In the third week, each mouse was first injected intraperitoneally with probenecid (250 mg/kg) once a day, and then injected with MPTP (25 mg/kg) once a day, for 5 days. In the blank group, each mouse was injected with only the corresponding solvent in the first three weeks. In the fourth week, each mouse in the blank group and the PD-ctrl group was administered with 0.1% DMSO solution by gavage every day, while each mouse in the PD-PAO group was administered with PAO at a dose of 0.3 mg/kg (by dissolving PAO in the 0.1% aqueous DMSO solution) by gavage every day, for 7 days.

All mice underwent fasting for solids but were supplied with water on day 6 of the fourth week. On day 7, the preference for sugar water experiment was carried out. As shown in FIG. 8A, the mice in the blank group have a clear preference for sugar water, the mice in the PD-ctrl group have a preference for sugar water significantly reduced (One way ANOVA, p<0.05), and the mice in the PD-PAO group still have a preference for sugar water, which is not significantly different from the blank group. After the sugar water preference experiment, the injection and administration by gavage were stopped in each group. All mice underwent fasting for solids but were supplied with water on day 6 of the sixth week. On day 7, the sugar water preference experiment was carried out. As shown in FIG. 8B, all mice in the three groups have a clear preference of sugar water with no significant difference from each other.

During the above experiment, 12 mice died in the PD-ctrl group, 6 mice died in the PD-PAO group, and there was no death in the blank group.

### 3.3 PAO and its derivatives inhibit anxiety behavior in wild-type mice

42 male ICR mice aged 3 months were randomly divided into two groups. The 22 mice in the first group were administered with a placebo (0.1% aqueous DMSO solution) by gavage every day, as a placebo group (vehicle), for 12 days. The other mice in the second group were administered with PAO with 0.1% aqueous DMSO solution at a dose of 0.3 mg/kg weight by gavage every day, as a PAO-treated group, for 12 days. On day 12, the NSF test was carried out. 24 hours before the NSF test, all mice underwent fasting for solids but were supplied with water. As shown in FIG. 9A, after entering the new environment, the mice in the placebo group need an average of 201.6±21.5 s (n=22) to start eating, while the mice in the PAO-treated group only need an average of 159.6±20.3 s (n=20), which is significantly shorter than the placebo group (T Test, p<0.05). Therefore, PAO can inhibit the anxiety behavior in wild-type mice in a new environment.

Another 47 ICR mice aged 3 months were randomly divided into three groups. The 16 mice in the first group were administered with a placebo (0.1% aqueous DMSO solution) by gavage every day, as a placebo group (vehicle), for 7 days. The 15 mice in the second group were administered with PI05 with 0.1% aqueous DMSO solution at a dose of 0.3 mg/kg weight by gavage every day, as a PI05-treated group, for 7 days. The 16 mice in the third group were administered with PI06 with 0.1% aqueous DMSO solution at a dose of 0.3 mg/kg weight by gavage every day, as a PI06-treated group, for 7 days. On day 7, the NSF test was carried out. 24 hours before the NSF test, all mice underwent fasting for solids but were supplied with water. As shown in FIG. 9B, after entering the new environment, the mice in the placebo group need an average of 216.6±21.2 s (n=16) to start eating, while the mice in the PI05-treated group and the PI06-treated group respectively need an average of 194.6±21.7 s (n=15) and an average of 154.2±21.2 s (n=16). The PI06-treated group has a feeding latency significantly reduced compared with the placebo group (one way ANOVA, p<0.05), and the PI05-treated group shows a tendency of reduced feeding latency.

Therefore, PAO and its derivatives can inhibit the anxiety behavior in wild-type mice in a new environment.

### Example 4: Effects of inhibition of PI4KIIIα, TTC7, Hyccin, and RBO on the expression of PI4KIIIα complex

### Materials and Method

### Drosophila strains and genetics

Emergent Drosophila adults were cultured in a standard Drosophila medium with a 12/12 hour light/dark cycle at 25°C.

The Drosophila strains used include: *rbo*²/Cyo-GFP (*rbo²* is a knockout mutation of the *rbo* gene, Cyo-GFP is a balancer chromosome expressing green fluorescent protein (GFP), *rbo*^{*-*/*+*}), *rbo²*/*rbo²;rbo-egfp*/*rbo-egfp* (a mutant of homozygote for the *rbo²* mutation and homozygote for the *rbo-egfp* transgene, the Drosophila strain is referred to as *rbo-gfp* for short) (the *rbo-egfp* transgene is a recombinant DNA constructed from a genomic DNA fragment containing complete *rbo,* and a DNA sequence encoding the green fluorescent protein (GFP) is inserted in front of the stop codon of *rbo,* and therefore the *rbo-egfp* transgene expresses the RBO-GFP fusion protein), *P[lacW]l(2)k14710*^{k15603}/Cyo-GFP (*ttc7* gene mutant formed by the insertion of transposon P[lacW], also referred to as *ttc7*^{*-*/*+*}), 104577/Cyo-GFP (104577 is a chromosomal mutation resulting from the deletion of the hyccin gene and its surrounding DNA segment, also referred to as *hyccin*^{*-*/*+*}), *pi4k*^{*GS27*/}*⁺, pi4k*^{*FY24*/}*⁺, pi4k*^{*FQ88*/}*⁺,* and *pi4k*^{*GJ86*/*+*} ( four heterozygotes for nonsense mutations of the gene encoding PI4KIIIα protein, all have balancer chromosomes FM7 and Gr-GFP that express the GFP, and are constructed by Schupbach's laboratory at Princeton University, USA).

### Western blotting and co-immunoprecipitation experiments

500 (for co-immunoprecipitation) and 100 (for western blotting) Drosophila heads of each genotype were collected and treated uniformly. The Drosophila heads were placed on ice, lysed with a pre-chilled Tris buffer containing 1% NP40 and a protease inhibitor, and then homogenized. The co-immunoprecipitation experiment was carried out according to the instructions of the Pierce^{®} Co-Immunoprecipitation (Co-IP) kit (Thermo Scientific). The products from the co-immunoprecipitation experiment were separated using SDS-PAGE gels and stained with Coomassie brilliant blue. The primary antibodies used include: anti-GFP (Casico), anti-RBO and PI4KIIIα (Zhang et al., J Neurosci., 2017, pp. 4928-4941), anti-Hyccin (produced by Abmart (Shanghai) using the FAM126 fragment RLPPIKNPRQ as antigen), anti-neuroglin (DSHB, clone BP104), anti-β-actin, and anti-Tubulin (Cell Signaling).

### Isolation of PM fragments from tissue lysate

The Drosophila brain tissue was placed on ice and treated with a pre-chilled Tris buffer containing 1% NP40 and a protease inhibitor. Then, the lysate was collected and centrifuged at 15000 g at 4°C for 1 h. The supernatant was transferred to a new EP tube, and the precipitate was resuspended with the same Tris buffer as above. Both the supernatant and resuspended precipitate were subjected to isolation with SDS-PAGE gels and western blotting.

### Statistical analysis

Data analysis was carried out using the software GraphPad Prism. All data are presented as mean ± SEM and statistically significant at p<0.05 (^{∗}p<0.05, ^{∗∗}p<0.01, and ^{∗∗∗}p<0.001).

### Results

**PI4KIIIα, TTC7, Hyccin, and RBO interact on the cell membrane to form a protein complex-PI4KIIIα complex, which are all indispensable parts of the PI4KIIIα complex.**

It can be shown from Coomassie brilliant blue staining in SDS-PAGE gel electrophoresis (FIG. 10A) that: the anti-GFP antibody can co-immunoprecipitate PI4KIIIα, TTC7, Hyccin, and RBO-GFP proteins from a homogenate of Drosophila heads expressing the RBO-GFP fusion protein (*rbo-gfp*)*,* but not from a homogenate of Drosophila heads that does not express the RBO-GFP fusion protein. PI4KIIIα, TTC7, and Hyccin proteins can be identified from the proteins co-immunoprecipitated with the RBO-GFP fusion protein by protein mass spectrometry (table 10). Consistent with this, it can be analyzed by protein mass spectrometry that the anti-RBO antibody can co-immunoprecipitate PI4KIIIα, TTC7, Hyccin, and RBO proteins from a homogenate of wild-type Drosophila heads (Table 11); and it can be confirmed from western blotting in SDS-PAGE gel electrophoresis that the PI4KIIIα, Hyccin, and RBO proteins co-immunoprecipitate with each other (FIG. 10B).

It can be shown from western blotting in SDS-PAGE gel electrophoresis (in FIG. 10C) that: the RBO protein is only expressed on the cell membrane, while the Hyccin protein is expressed on both the cell membrane and the cytoplasm, but mainly located in the cytoplasm; the knockout of the *hyccin, rbo,* or *ttc7* gene leads to the deletion of RBO; and the knockout of the *rbo* or *ttc7* gene also leads to the deletion or significant reduction of Hyccin protein in the cell membrane, but does not significantly change the expression level of Hyccin protein in the cytoplasm. This indicates that the PI4KIIIα, TTC7, Hyccin, and RBO proteins on the cell membrane are interdependent in the expression, and are all indispensable parts of the PI4KIIIα complex. However, the Hyccin expression in the cytoplasm is independent of TTC7 and RBO.

It can be shown from western blotting in SDS-PAGE gel electrophoresis (in FIG. 10D) that: the knockout of one copy of *hyccin* can significantly reduce the total expression level of Hyccin, but the knockout of one copy of *rbo* does not change the total expression level of Hyccin.

It can be shown from western blotting in SDS-PAGE gel electrophoresis (in FIG. 10E) that: the knockout of one copy of *PI4KIIIα* can also significantly reduce the expression level of RBO, further indicating the interdependence of the components of the PI4KIIIα complex.

**Table 10. Mass spectrometry analysis of specific RBO-GFP-interacting proteins co-immunoprecipitated with GFP antibody in homogenates of rbo-gfp and wt Drosophila**

| | PepCount | CoverPercent | MW (kd) |
|---|---|---|---|
| RBO-GFP | 98 | 52.3% | 122 |
| PI4KIII | 186 | 35.5% | 244 |
| TTC7 | 77 | 43.1% | 95 |
| Hyccin | 33 | 32.7% | 70 |

**Table 11. Mass spectrometry analysis of RBO-interacting proteins co-immunoprecipitated with RBO antibody (not control protein) in a homogenate of wild-type Drosophila**

| | PepCount | CoverPercent | MW (kd) |
|---|---|---|---|
| RBO | 93 | 44.8% | 95 |
| PI4KA | 215 | 37.8% | 244 |
| TTC7 | 55 | 30.6% | 95 |
| Hyccin | 20 | 25.4% | 70 |

## Claims

1. A method for preventing or treating a mood disorder, comprising administering an effective amount of PI4KIIIα/FAM126/TTC7 complex inhibitor to a subject in need thereof.

2. The method according to claim 1, wherein the PI4KIIIα/FAM126/TTC7 complex inhibitor is a small-molecule compound, an antibody, an RNAi molecule, or an antisense nucleic acid.

3. The method according to claim 1, wherein the PI4KIIIα/FAM126/TTC7 complex inhibitor is an EFR3-specific inhibitor.

4. The method according to claim 3, wherein the EFR3-specific inhibitor is an EFR3a-specific inhibitor or an EFR3b-specific inhibitor.

5. The method according to claim 1, wherein the PI4KIIIα/FAM126/TTC7 complex inhibitor is a PI4KIIIα-specific inhibitor.

6. The method according to claim 5, wherein the PI4KIIIα-specific inhibitor is a small-molecule compound.

7. The method according to claim 2 or 6, wherein the small-molecule compound has a structure of formula (I) or a pharmaceutically acceptable salt thereof, 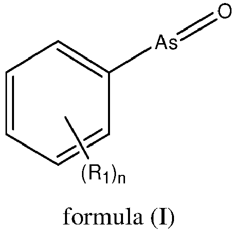 wherein R₁ is independently selected from
(a) H, deuterium, halogen, nitro, cyano, hydroxyl, amino, carbamoyl, C₁₋₆ alkyl, C₂₋₆ alkynyl, C₂₋₆ alkenyl, C₁₋₆ alkoxy, C₁₋₆ haloalkyl, C₁₋₆ alkylene-NH₂, C₁₋₆ alkylene-NH-C(O)H, -As(O), -N=NH, -NH-(C₁₋₆ alkyl), N,N-(C₁₋₆ alkyl)₂, -NH-C(O)H, -NH-S(O)₂H, -C(O)OH, -OC(O)H, -SH, -S(O)₂H, -S(O)₂-NH, or heterocyclyl, which is optionally substituted by R₂ or R₃, wherein R₂ and R₃ are each independently selected from amino, C₁₋₆ alkyl, C₁₋₆ alkoxy, C₁₋₆ haloalkyl, - NH-(C₁₋₆ alkyl), -NH-(6- to 12-membered aryl), -N,N-(C₁₋₆ alkyl)₂, C₃₋₆ cycloalkyl, 6- to 12-membered aryl, or 3- to 12-membered heterocyclyl, which is optionally substituted by one or more of halogen, nitro, cyano, hydroxyl, amino, carbamoyl, -NH-C(O)-R₅, -C(O)OR₄, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-, and R₄ is C₁₋₆ alkyl, which is optionally substituted by one or more of halogen, nitro, cyano, hydroxyl, amino, carbamoyl, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-, and R₅ is selected from H, C₁₋₆ alkyl, C₂₋₆ alkynyl, C₂₋₆ alkenyl, C₁₋₆ alkoxy, or C₁₋₆ haloalkyl, or
(b) R₁ on two adjacent carbon atoms forms 5- to 12-membered cycloalkyl, aryl, or heterocyclyl, which is optionally substituted by one or more of halogen, nitro, cyano, hydroxyl, amino, carbamoyl, 6- to 12-membered aryl, C₁₋₆ alkyl, C₂₋₆ alkynyl, C₂₋₆ alkenyl, C₁₋₆ alkoxy, C₁₋₆ haloalkyl, 3- to 6-membered heterocyclyl, C₃₋₆ cycloalkyl, or Bn-O-,
wherein n is an integer from 0 to 5.

8. The method according to claim 7, wherein R₁ is each independently selected from H, deuterium, halogen, nitro, cyano, hydroxyl, amino, carbamoyl, C₁₋₆ alkyl, C₁₋₆ alkoxy, C₁₋₆ haloalkyl, -As(O), -NH-(C₁₋₆ alkyl), -N,N-(C₁₋₆ alkyl)₂, or -C(O)OR₆, wherein n is an integer from 0 to 2, and R₆ is C₁₋₆ alkyl.

9. The method according to claim 7, wherein R₁ is each independently selected from H, deuterium, halogen, nitro, cyano, hydroxyl, amino, C₁₋₆ alkyl, C₁₋₆ alkoxy, C₁₋₆ haloalkyl, or - As(O), wherein n is an integer from 0 to 2.

10. The method according to claim 7, wherein R₁ is each independently selected from H, deuterium, halogen, amino, C₁₋₆ alkoxy, or C₁₋₆ haloalkyl, wherein n is 1.

11. The method according to any one of claims 7 to 10, wherein R₁ is located at an ortho position and/or para position of the -As(O) group.

12. The method according to claim 11, wherein n is 0.

13. The method according to claim 6, wherein the small-molecule compound is selected from the group consisting of the following compounds:

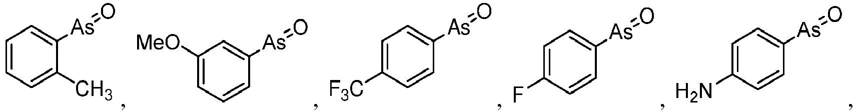

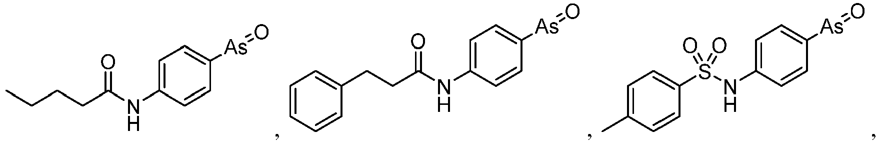

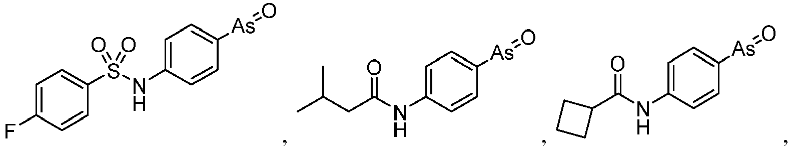

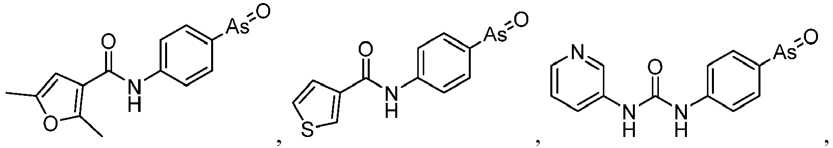

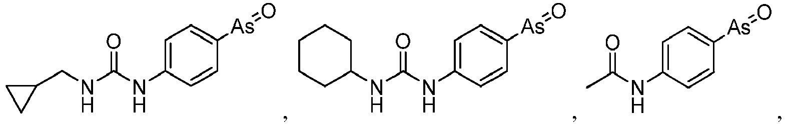

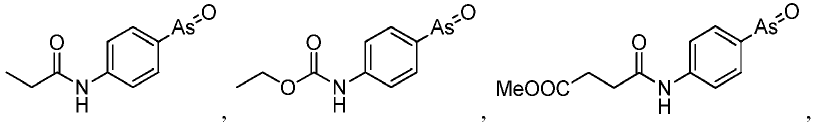

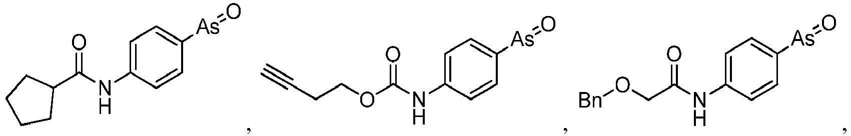

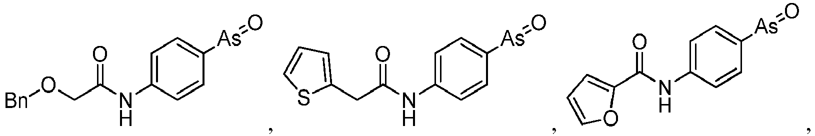

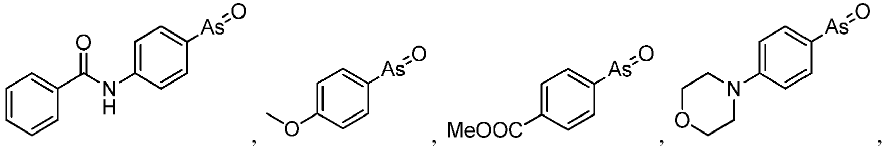

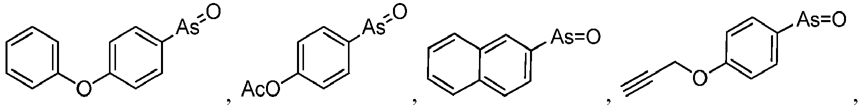

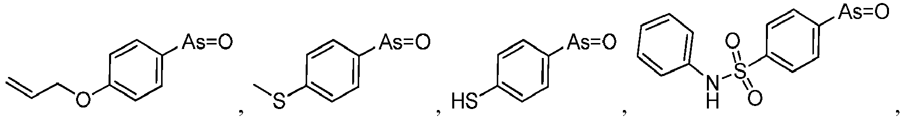

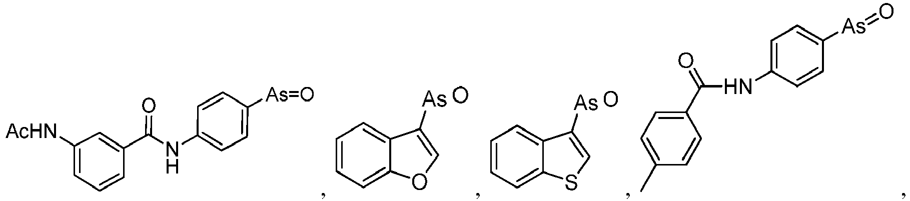

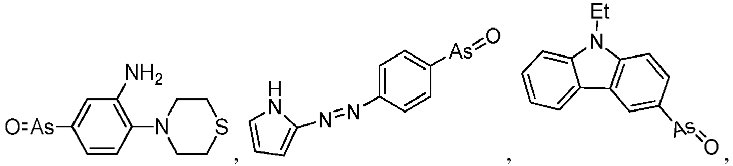

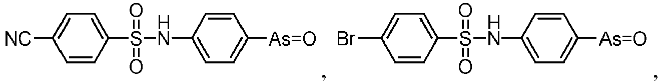

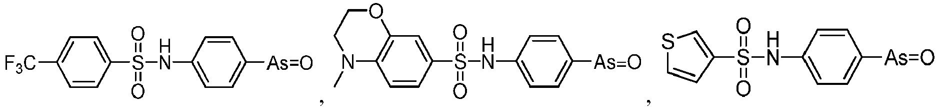

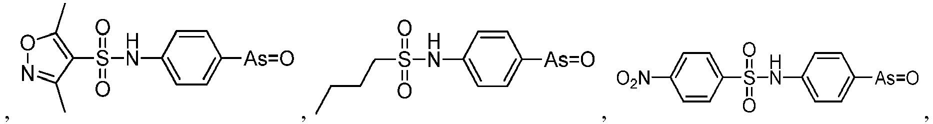

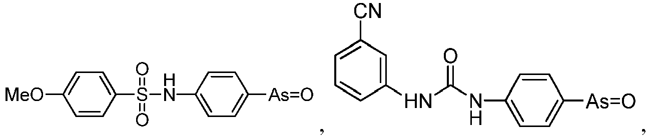

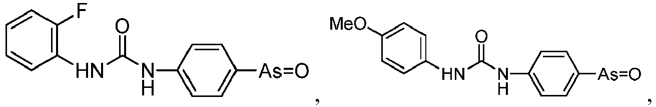

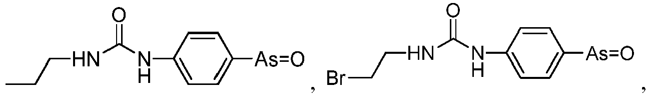

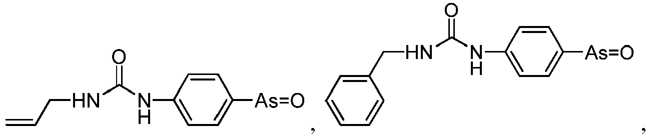

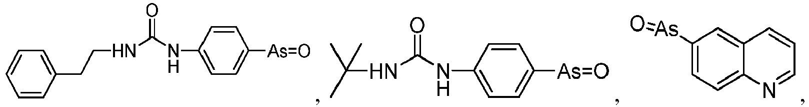

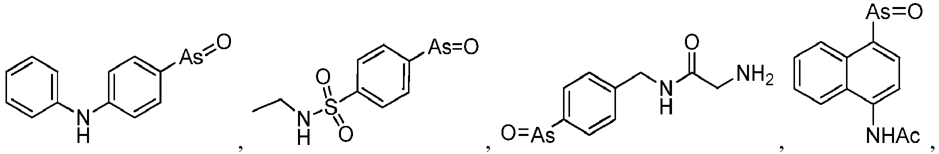

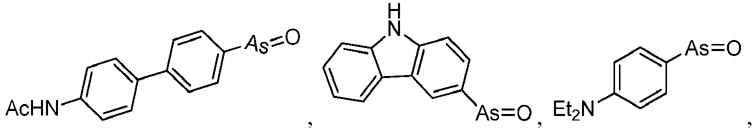

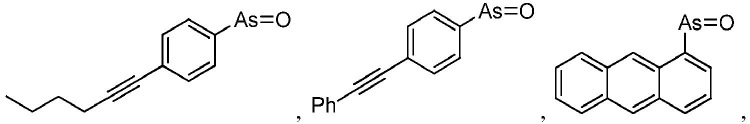

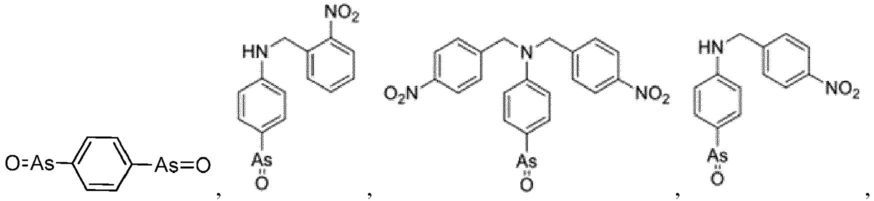

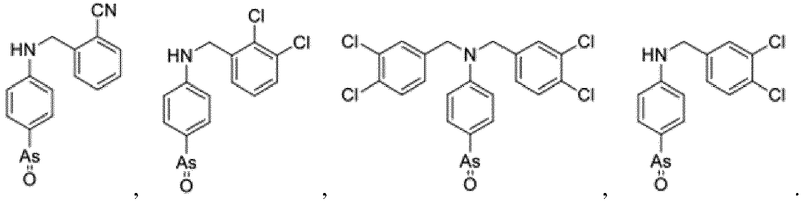

14. The method according to claim 2, wherein the antibody is a monoclonal antibody or a polyclonal antibody.

15. The method according to claim 2 or 14, wherein the antibody is a chimeric antibody, a humanized antibody, or a fully human antibody.

16. The method according to claim 2, wherein the RNAi molecule is a small interfering RNA (siRNA), a short hairpin RNA (shRNA), or a microRNA (miRNA).

17. The method according to claim 2 or 16, wherein the RNAi molecule has a length of 18-100 bases.

18. The method according to any one of claims 2, and 16-17, wherein the RNAi molecule is modified to enhance its stability.

19. The method according to claim 1, wherein the subject is a human or a mammal.

20. The method according to claim 1, wherein the mood disorder is an elevated mood, a depressed mood, or a cycle of elevated and depressed moods.

21. The method according to claim 1, wherein the mood disorder is anxiety, depression, schizophrenia, or mania.

22. The method according to claim 1, wherein the mood disorder is caused by a neurodegenerative disease, stroke, or malignant tumor.

23. The method according to claim 1, wherein the mood disorder is anxiety or depression caused by a neurodegenerative disease.

24. The method according to claim 1, wherein the PI4KIIIα/FAM126/TTC7 complex inhibitor is administered orally, subcutaneously, intramuscularly, or intravenously.

25. The method according to claim 1, further comprising diagnosing the subject as having the mood disorder before administering an effective amount of PI4KIIIα/FAM126/TTC7 complex inhibitor to the subject.

26. The method according to claim 1, further comprising administering a second reagent to the subject in need thereof.

27. The method according to claim 26, wherein the second reagent is a reagent used for treating the mood disorder.

28. The method according to claim 26, wherein the second reagent is reagent used for treating a neurodegenerative disease, stroke, or malignant tumor.

29. The method according to claim 26, wherein the PI4KIIIα/FAM126/TTC7 complex inhibitor is administered before, after, or simultaneously with the second reagent.

30. Use of a PI4KIIIα/FAM126/TTC7 complex inhibitor in manufacture of a medicament for preventing or treating a mood disorder.

31. Use of a PI4KIIIα/FAM126/TTC7 complex inhibitor in preventing or treating a mood disorder, comprising administering an effective amount of PI4KIIIα/FAM126/TTC7 complex inhibitor to a subject in need thereof.
